(19) Europäisches Patentamt
European Patent Office
Office européen des brevets

(11) EP 1 627 664 A1

# (12) EUROPEAN PATENT APPLICATION

(43) Date of publication:
**22.02.2006 Bulletin 2006/08**

(51) Int Cl.:
***A61Q 5/06*** (2006.01) ***A61K 8/81*** (2006.01)

(21) Application number: **05254784.1**

(22) Date of filing: **29.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **30.07.2004 US 592887 P**
**25.07.2005 US 189004**

(71) Applicant: **L'OREAL**
**75008 Paris (FR)**

(72) Inventors:
- **Quadir, Murat**
**Scotch Plains, New Jersey 07076 (US)**
- **Rollat-Corvol, Isabelle**
**Paris 75017 (FR)**
- **Burakov, Dina**
**Edison, New Jersey 08837 (US)**

(74) Representative: **Benson, John Everett**
**J. A. Kemp & Co.,**
**14 South Square,**
**Gray's Inn**
**London WC1R 5JJ (GB)**

## (54) Hair styling composition comprising adhesive particles and non-adhesive particles

(57) The invention relates to a hair styling composition comprising: an effective amount of at least one adhesive particle to provide a reshapable effect, an effective amount of at least one polymeric non-adhesive particle to impart at least one of the desirable properties and/or substantially maintain said reshapable effect relative to an identical formulation without said at least one non-adhesive particles, and a cosmetically acceptable carrier. The invention also relates to a method of providing a reshapable effect to the hair, comprising applying to the hair, before, during or after shaping of the hairstyle, a hair styling composition of according to the invention. And finally, the invention also relates to a method of making a reshapable hair styling composition.

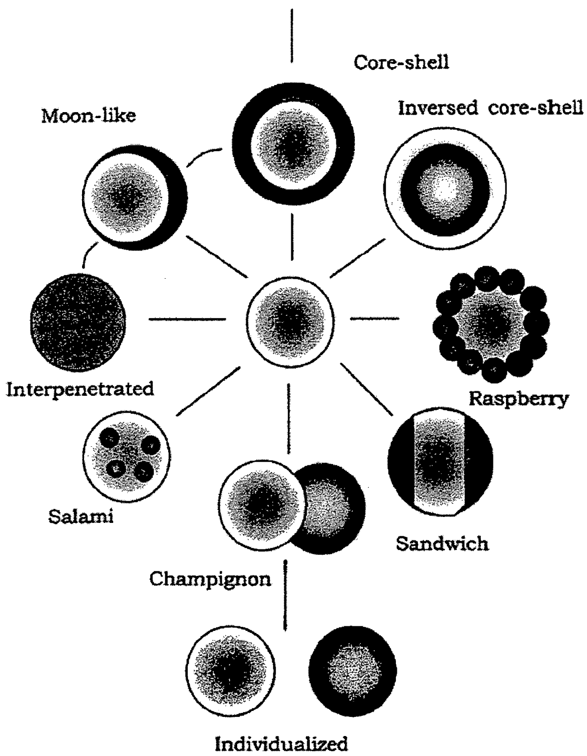


FIGURE 1

EP 1 627 664 A1

## Description

This application claims the benefit of the filing date of United States Provisional Patent Application No. 60/592,887 filed July 30, 2004, the disclosure of which is hereby incorporated herein by reference.

The present invention relates to compositions and methods for styling hair. More particularly, the invention relates to hair styling compositions and methods that, in preferred embodiments, enable repeated fixing and shaping of hair without reapplication of hair styling compositions or heat.

### BACKGROUND OF THE INVENTION

Fixing hair in place is an important element in hair styling, and this generally involves either fixing a shape that has already been styled, or simultaneously shaping and fixing of the hair.

There are countless products available to consumers to style his/her hair in a particular shape or configuration and to maintain the desired shape or configuration intact. Hold is usually accomplished by the delivery of a composition containing a film former and/or an adhesive that remains on hair after evaporation of some or all of the carrier.

While the hair styling compositions currently on the market are very effective in holding the hair in a desired shape or configuration, many are not designed to allow the hairstyle to be later modified to a desired shape or reshaped into the original style without reapplying a hair styling composition and/or heat. Moreover, under various kinds of stress, hairstyles resulting from the use of conventional products have a tendency to take on an undesirable permanent set, which cannot easily be modified.

The invention described in U.S. Patent No. 6,548,051 overcomes the above shortcomings of traditional hair styling composition by providing a reshapable hair styling composition. The patent discloses a hair styling composition comprising, optionally in a cosmetically acceptable carrier, at least one adhesive particle, wherein the at least one adhesive particle comprises at least one substrate and at least one partial coating, wherein the at least one adhesive particle is present in an amount effective to obtain a reshapable effect. This patent also describes a method of providing a reshapable effect to the hair by applying to the hair reshapable hair styling composition explained above.

U.S. Patent Application Publication No. 2002/0059941 also describes a hair styling composition that provides a reshapable hair styling composition. The application discloses a hair styling composition that comprises at least one encapsulated adhesive, optionally in a cosmetically acceptable carrier, wherein the at least one encapsulated adhesive comprises at least one adhesive encapsulated by at least one encapsulating material.

While there have been pioneering advances in hair styling products by the introduction of these reshapable hair care systems, further improvement remains desirable. Further advances in adjusting the degree of adhesion and improving the overall look, feel and manageability of hair are still worthy, if not lofty goals.

### SUMMARY OF THE INVENTION

Unexpectedly, it has now been found that a composition can be produced that provides significantly improved hair styling properties. In one embodiment, the present invention allows one to modify and/or adjust the adhesive properties of a reshapable hair care system by the concurrent use of both adhesive and non-adhesive particles in hair styling products.

In another embodiment, the present invention provides formulations that are effective in holding the hair in a desired shape or configuration. In a more particularly preferred embodiment, the present invention also permits repeated fixing into different styles and/or reshaping of hair. In one desirable aspect of the invention, this is accomplished without reapplication of additional hair styling compositions or heat.

In certain embodiments, the present invention facilitates imparting at least one desirable property such as, without limitation, reduced tactile stickiness, luster, shine, silky feel or appearance to hair, particularly hair treated with products containing adhesive particles. In certain other embodiments, the present invention facilitates substantially maintaining the reshapability of hair to which it is applied. In a particularly preferred embodiment, the present invention provides formulations that facilitate imparting at least one desirable property such as, without limitation, reduced tactile stickiness, luster, shine, silky feel and/or appearance, while substantially maintaining the hair's fixability and reshapability.

In one embodiment, the present invention comprises at least one non-adhesive particle and at least one adhesive particle, optionally in a cosmetically acceptable carrier. The presence of the at least one non-adhesive particle, when properly selected and provided in effective amounts relative to the adhesive particles, can significantly reduce at least one of the sometimes undesirable properties that can be realized by use of some reshapable systems such as, without limitation, tacky, sticky feel or dull appearance of hair. Reduction is measured by the application of these products when compared to the application of an identical formulation without the non-adhesive particles as judged by hair stylists. Moreover, the presence of the at least one non-adhesive particle when properly selected and provided in effective amounts relative to the amount of adhesive particles, can substantially maintain the fixability and reshapability (collectively

the "reshapable effect") of hair and a hairstyle, again when compared to an identical formulation without the non-adhesive particles and again in the judgment of hair stylists. The hair style may even remain reshapable, with normal use for about 24 hours or more.

In one embodiment, the present invention is especially suitable for providing "acceptable results" in terms of imparting or lessening the degree of reduction in at least one desirable property such as, without limitation, greasiness, stickiness, tackiness, smoothness, silkiness or appearance, to the hair when compared to an identical formulation without the non-adhesive particles judged by professional stylists. In yet another embodiment, the hair styling composition both substantially maintains a reshapable effect and provides acceptable results.

In one embodiment, the present invention provides hair styling compositions or formulations including at least one adhesive particle (a term which includes encapsulated adhesive particles, non-encapsulated adhesive particles or a mixture thereof) and at least one non-adhesive particle. In one embodiment, the present invention provides hair styling compositions or formulations including at least one adhesive particle (a term which includes encapsulated adhesive particles, non-encapsulated adhesive particles or a mixture thereof) and at least one non-adhesive particle in an amount that is effective to reduce at least one undesirable property such as, without limitation, greasy, tacky, sticky feel or dull appearance of hair while substantially maintaining hold or the reshapable effect when compared to an identical formulation without the non-adhesive particles.

Another aspect of the present invention is a hair styling composition including both adhesive and non-adhesive particles. The adhesive particles can be provided in an effective amount of at least up to about 15% by weight of the formulation. In another embodiment, the adhesive particles are provided in an amount of between about 0.05% to about 15% by weight of the finished formulation or product and in yet another embodiment, between about 0.05% and about 3.0%. In still another embodiment, the adhesive particles are provided in an amount of between about 0.05% and about 2.0% by weight. In another embodiment, the non-adhesive particles can be provided in an effective amount of up to about 10% by weight of the formulation or product. In yet another embodiment, the non-adhesive particles are provided in an amount of between about 0.03% to about 2.0% by weight of the finished formulation or product and in yet another embodiment, between about 0.04% and about 0.3%. In still another embodiment, the non-adhesive particles are provided in an amount of between about 0.04% and about 0.2% by weight.

In an embodiment, the adhesive particles are provided in an amount that is equal to or greater than the amount of the non-adhesive particles. In another embodiment, the ratio of adhesive to non-adhesive particles is 1:1 to 250:1. In another embodiment, the ratio is from about 1:1 to about 100:1. In still another embodiment, the ratio is from about 2:1 to about 50:1 and in yet another embodiment from about 5:1 to about 45:1, and in still another embodiment, from about 10:1 to about 25:1. These ratios are particularly, but not exclusively, useful for mousse formulations.

In yet another embodiment, the composition is a gel and the adhesive particles are provided in an amount from between about 0.05% to about 15% by weight of the gel formulation and the non-adhesive particles are provided in an amount of from about 0.03% to about 2.0%, more preferably from about 0.04% to about 0.3% by weight of the gel formulation and in still a more preferred embodiment, the non-adhesive particles are present in an amount of about 0.04% to about 0.2% by weight of the gel.

In another embodiment, the composition is a mousse having from about 0.1% to about 5% adhesive particles and from about 0.03% to about 2.0% non-adhesive particles by weight of the mousse formulation, more preferably from about 0.04% to about 0.3% by weight of the gel formulation and in still a more preferred embodiment, the non-adhesive particles are present in an amount of about 0.04% to about 0.2% by weight of the mousse.

In another embodiment of the invention, the non-adhesive particles are polymeric and have a core/shell structure. Particularly preferred materials for the core/shell particles are polystyrene and polymethyl methacrylate, where either is used as the core or shell material. Even more preferably, polystyrene is the core material, and polymethyl methacrylate is the shell material.

In yet another embodiment, the non-adhesive particles of the invention are inorganic. Mixtures of any of the previously discussed polymeric particles with such inorganic particles, in any proportion, are also contemplated.

In yet another embodiment of the invention, a blend of non-adhesive particles of different polymeric materials (at least one of which is not a core/shell structure), with or without inorganic non-adhesive particles, may be used. Representative polymeric materials include, but are not limited to, the same materials used in the aforementioned core/shell particles, such as polystyrene, and polymethyl methacrylate. The blend may include a mixture of two or more different types of polymer particles.

In another embodiment of the invention, the at least one non-adhesive particle is constituted of a single polymeric material (not in a core/shell structure). These may or may not be mixed with inorganic particles. Representative materials include polystyrenes and polyacrylates.

In yet another embodiment, the non-adhesive particles are substantially all inorganic particles.

In still another embodiment of the invention, a blend of non-adhesive particles which may be selected from inorganic particles, core/shell structured particles, polymeric particles without a core/shell structure, or a mixture of core/shell and non-core/shell structure polymeric materials, with or without inorganic non-adhesive particles, may be used.

In one embodiment, the non-adhesive particles have an average particle size of about 6 to about 30 microns, and in another embodiment from about 7 to about 25 microns. In another embodiment, the non-adhesive particles have an average particle size of about 8 to about 20 microns when polymeric. These non-adhesive particles may have an aspect ratio of about 1.2 or less, preferably from about 1.0 to about 1.2. In one embodiment, these particles are cross-linked polymers having a glass transition temperature of about 50 degrees Celsius or more. When the non-adhesive particles are inorganic, they may have an average particle size of about 2 to about 80 microns, preferably about 8 to about 70 microns, more preferably about 15 to about 60 microns and/or an aspect ratio of about 1.2 or less, preferably from about 1.0 to about 1.2.

In yet another embodiment, the particles used, both adhesive and non-adhesive, need not be spherical. Rather, they can be doughnut shaped, hemispheric, rod-like, or even irregular.

Methods of shaping and/or reshaping hair using the compositions of the present invention and methods of making these compositions and methods of their application are also contemplated.

In one embodiment, the present invention provides a hair styling composition comprising: an effective amount of at least one adhesive particle of up to about 15% by weight of the composition. The composition also comprises an effective amount of at least one non-adhesive particle selected from a single type of polymer particles, a blend of different polymer particles or core/shell polymer particles, or inorganic non-adhesive particles, provided in an amount of up to about 2% by weight of the composition. The compositions also include at least one cosmetically acceptable carrier. The hair styling composition provides a reshapable effect, e.g. the composition is a reshapable hair styling composition which permits styling or reshaping a hair style even 24 hours after application, preferably without the need for additional products or heat during reshaping or restyling.

In a preferred embodiment, the hair styling formulation is a gel or mousse.

The invention also provides a hair styling composition comprising: an effective amount of at least one adhesive particle to provide a reshapable effect, an effective amount of at least one non-adhesive particle to impart at least one of the desirable properties and/or substantially maintain said reshapable effect relative to an identical formulation without said at least one non-adhesive particles, and a cosmetically acceptable carrier.

The present invention also provides and aerosol hair styling composition comprising: an effective amount of at least one adhesive particle sufficient to provide a reshapable effect, preferably up to about 15% by weight. Also provided is an effective amount of at least one polymeric or inorganic non-adhesive particle of up to about 10% by weight and a vaporizable solvent and a propellant. In a particularly preferred embodiment of this aspect of the present invention, the propellant is provided in an amount of less than 10% by weight based on the weight of the composition, more preferably about 7% or less, and even more preferably about 6% or less. In a particularly preferred embodiment of this aspect of the present invention, the propellant is provided in an amount of from about 0.1% to 10% by weight based on the weight of the composition, more preferably from about 0.1% to 7%, and even more preferably from about 0.1% to about 6%. Solvents include, without limitation, water and lower alcohols. Propellants include, without limitation, air, carbon dioxide, dimethyl ether, halogenated hydrocarbons including fluorinated or non-fluorinated hydrocarbons.

The present invention also provides and aerosol hair styling composition comprising: an effective amount of at least one adhesive particle to provide a reshapable effect, an effective amount of at least one non-adhesive particle to impart at least one of the desirable properties and/or substantially maintain said reshapable effect relative to an identical formulation without said at least one non-adhesive particles, a vaporizable solvent and a propellant. In a particularly preferred embodiment of this aspect of the present invention the propellant is provided in an amount of from about 0.1% to 10% by weight based on the weight of the composition, more preferably from about 0.1% to 7%, and even more preferably from about 0.1% to about 6%.

In another embodiment, the present invention provides an aerosol hair styling composition comprising: at least one adhesive particle in an amount of up to about 15% by weight of the composition. The composition also comprises at least one non-adhesive particle selected from a single type of polymer particles, a blend of different polymer particles, inorganic particles or core/shell polymer particles, and mixtures thereof, provided in an amount of up to about 2% by weight of the composition. In a particularly preferred embodiment of this aspect of the present invention the propellant is provided in an amount of from about 0.1% to 10% by weight based on the weight of the composition, more preferably from about 0.1% to 7%, and even more preferably from about 0.1% to about 6%.

In a particularly preferred embodiment, these aerosol compositions are provided in an aerosol container and the invention also contemplates the application of these compositions by spraying an effective amount of an aerosol composition as described herein onto the hair of a subject from an aerosol container.

In yet a further embodiment, the present invention provides a reshapable hair composition comprising or consisting essentially of up to about 16.5% total of adhesive and inorganic non-adhesive particles, from about 0.05% to about 15% of at least one adhesive particle by weight based on the weight of the composition, from about 0.2% to about 12% of at least one inorganic non-adhesive particle by weight of the adhesive particles and at least one carrier.

In a particularly preferred aspect of the present invention, there is provided a hair styling composition, which is a mousse or a gel, which comprises a mixture of an effective amount of at least one adhesive particle of up to about

10% by weight of the composition, an effective amount of at least one polymeric non-adhesive particle selected from a blend of different particles and core/shell particles in an amount of up to 5% by weight of the composition, and at least one cosmetically acceptable carrier. In one aspect of this embodiment, the hair styling composition provides reshapability and specifically substantially maintains the reshapeable effect. In another aspect of this embodiment, the product imparts at least one of less tackiness, less stickiness, less greasiness, more silkiness, smoother feel or improved appearance when compared to an identical mixture without the non-adhesive particles.

In a particularly preferred embodiment in accordance with the present invention, the amount of adhesive particles is provided in an amount of up to about 2.5% by weight and the amount of non-adhesive particles is provided in an amount of up to about 1% by weight.

These and other embodiments of this invention will become apparent in light of the following disclosure.

BRIEF DESCRIPTION OF THE DRAWINGS

Figure 1 illustrates various types of core/shell non-adhesive polymeric particles, starting at the top and moving clockwise, including core/shell, inversed core/shell, raspberry, sandwich, champignion, salami, interpenetrated and moon-like.

DETAILED DESCRIPTION

While the specification concludes with the claims particularly pointing and distinctly claiming the invention, it is believed that the present invention will be better understood from the following description. All percentages and ratios used herein are by weight of the total composition and all measurements made are at 25°C and normal pressure unless otherwise designated. The present invention can comprise (open ended) or consist essentially of the components of the present invention as well as other ingredients or elements described herein. As used herein, "comprising" means the elements recited, or their equivalent in structure or function, plus any other element or elements which are not recited. The terms "having" and "including" are also to be construed as open ended unless the context suggests otherwise. As used herein, "consisting essentially of" means that the invention may include ingredients in addition to those recited in the claim, but only if the additional ingredients do not materially alter the basic and novel characteristics of the claimed invention. Thus, other additives which do not unduly detract from reshapability, shine, tacky feel, silkiness, luster or appearance are contemplated. Materials that can adversely affect these qualities may still be considered as falling within the scope of the invention as long as they are used in an amount which is insufficient to detract from the properties of the composition sufficiently to prevent its use as a practicable commercial product. Preferably, such additives will not be present at all or only in trace amounts. However, it may be possible to include up to about 10% by weight of materials that could materially alter the basic and novel characteristics of the invention as long as the utility of the compounds (as opposed to the degree of utility) is maintained. All ranges recited herein include the endpoints, including those that recite a range "between" two values. Terms such as "about," "generally," "substantially," and the like are to be construed as modifying a term or value such that it is not an absolute, but does not read on the prior art. Such terms will be defined by the circumstances and the terms that they modify as those terms are understood by those of skill in the art. This includes, at very least, the degree of expected experimental error, technique error and instrument error for a given technique used to measure a value.

The term "hair styling composition" or more simply "composition" relates to any mixture of ingredients that can be used to affect, create or maintain a hairstyle, including, for example, sprays, mousses, styling gels, leave-on conditioners, shampoos, conditioners, permanent waving compositions, hair care products, hair treatment products, and hair styling products. This term is often used interchangeably with "formulation" unless the context suggests otherwise.

The term "carrier" refers to any solvent or other cosmetically acceptable propellant or ingredient that is useful in solubilizing, dispersing or delivering the adhesive particles and/or a non-adhesive particles and other materials in the formulations.

The term "reshapable" means to provide a hairstyle (fixability) and/or hold that can be restored or modified without additional material or heat being applied. This does not mean that additional product and/or heat may not be applied. It may be desirable to add heat and/or additional styling compositions in terms of speed, ease of use, if hair becomes unduly wet or dirty, is excessively combed, brushed or manipulated, washed, or when hair is to be dramatically restyled. At least some of the compositions in accordance with the invention should be reshapable, as judged by a professional hair stylist of ordinary skill, for at least 4 hours and up to 24 hours or more after initial application. Preferably and merely for example, in order to restore or modify the hairstyle in case of "drooping" or loss of setting (dishevelment), no new materials, such as water or any form of fixing agent, or heat are required. Other terms, which may be synonymous with reshapable, include repositionable, remoldable, restyleable, rearrangeable, and remodellable. The term "reshapable" also means to provide a hairstyle that can retain or hold a desired shape or configuration until water, heat, time and/or physical contact destroys the desired shape or configuration.

The term "substantially maintain the reshapable effect" or words to that effect means to provide a hold (fixability) and reshapability that is very similar in terms of length in time as an identical formulation without the non-adhesive particles. For example, the efficacy of some of the preferred compositions of the present invention comprising both at least one adhesive particle and at least one non-adhesive particle can be substantially the same as that resulting from the same formulation with only the at least one adhesive particle. A reshapable effect of the present invention can last for at least 4 hours and preferably about 24 hours or more, giving rise to a durable styling effect. This is judged by a professional stylist of ordinary skill.

The term "acceptable results" means that upon application of a hair styling composition to hair, it imparts (or reduces the degree that such properties are compromised) at least one of the following desirable properties such as, without limitation, less greasy, less tacky, less sticky, smoother, silkier feel or appearance as perceived by a professional stylist of ordinary skill while substantially maintaining the reshapable effect. These are measured by comparing a formulation in accordance with the present invention including non-adhesive particles to an otherwise identical formulation without the non-adhesive particle as judged by hair stylists. Other terms, which may be synonymous with acceptable results, include satisfactory results, good results, and suitable results.

It is to be understood that both the foregoing general description and the following detailed description are exemplary and explanatory only and are not restrictive of the invention as claimed.

Typically, hair styling compositions utilize liquid adhesives that lose all of their tack upon evaporation of the carrier. In other words, the adhesive bond or weld point is set when the carrier evaporates and cannot be reset or repositioned. The use of adhesive particles as described herein provides a mechanism for the product to remain tacky or sticky, i.e., retain some adhesive and/or reshapable properties, following application and/or evaporation of the optional carrier. Consequently, the hair may be moved or repositioned by simple combing, brushing, or other styling techniques, and a bond may be re-formed or reset.

Some formulations that provide reshapability may be too adhesive and/or may leave at least one undesirable property such as, without limitation, greasy, tacky, sticky feel or dull appearance of hair in order to provide this reshapable effect.

Unexpectedly, it has been discovered that the use of non-adhesive particles with adhesive particles retains and/or enhances desirable qualities including the ability to reshape the hair without leaving at least one of the undesirable properties such as, without limitation, greasy, tacky, sticky feel or dull appearance relative to an identical formulation without the non-adhesive particles. In addition to the improved feel and/or appearance, it has also been discovered that the use of non-adhesive particles with adhesive particles can substantially maintain the reshapable effect relative to the same formulation without the non-adhesive particles. Thus the improved properties are not accompanied by an unacceptable reduction in the ability of the product to hold hair and provide reshapability. Indeed, one of the advantages of the use of non-adhesive particles is that one can adjust the adhesive properties imparted by the adhesive particles. Thus, the use of the invention also allows one to tailor the adhesive effect or properties of the formulation.

Without wishing to be bound by any particular theory of operation, it is thought that the non-adhesive particles attach to the adhesive on the adhesive particles on contact. This reduces the tacky surface area and provides reduced access to hair, modifying the degree of hair which can be bound. This results in more particles with optimal amount of tack which can provide more weld points to hold and reshape a hairstyle in a desired shape or configuration. The synergy of the adhesive and non-adhesive particles in the present invention provides a hair styling composition that substantially maintains the reshapable effect and/or impart at least one of the desirable properties such as, without limitation, less tacky, less sticky, less greasy, silkier, smoother feel or appearance.

NON-ADHESIVE PARTICLES

As used herein, a "non-adhesive" particle is a particle that is not coated with an adhesive as described in U.S. Patent No. 6,548,051 and U.S. Patent Application Publication No. 2002/0059941. More specifically, a non-adhesive particle of the invention is a discrete substrate that is not an adhesive and is not coated with an adhesive coating in whole or in part, and does not comprise an adhesive as a core or shell material when in a core/shell structure. Non-adhesive particles can be divided roughly into two categories based on their composition -- polymeric and inorganic.

It is not necessary that the term non-adhesive particle embrace particles of a particular shape or geometry. However, in one aspect, the non-adhesive particles in accordance with the present invention will generally be roughly spherical, having an aspect ratio of 1.2. or less, more preferably from about 1.0 to about 1.2. In another embodiment, however, they may be, for example only, rod shaped, doughnut shaped, hemispheric and even irregular. Particle size generally ranges from between about 6 to about 30 microns, more preferably 7 to about 25 microns, and most preferably about 8 to about 20 microns for the polymeric based non-adhesive particles. For the inorganic non-adhesive particles, the size generally ranges from about 2 to about 80 microns, more preferably about 8 to about 70 microns and most preferably about 15 to about 60 microns. Particle size in accordance with the present invention for the polymeric particles means mean particle size by volume as measured by a Coulter multisizer.

Furthermore, the term particle does not imply that the non-adhesive particles in accordance with the present invention are solid, as they may be liquid, semisolid, gel, and the like.

The amount of non-adhesive particles useful in accordance with the present invention is, in one embodiment, an amount which is effective to or sufficient to, when mixed with an effective amount of adhesive particles, impart or enhance at least one of the desirable properties, such as, without limitation, less tacky, less sticky, less greasy, silkier, smoother feel or appearance and/or substantially maintain the reshapable effect. These are measured by comparing a formulation in accordance with the present invention including non-adhesive particles to an otherwise identical formulation including adhesive particles only as judged by professional stylists of ordinary skill. Any amount of non-adhesive particles that can provide these effects in accordance with the present invention is therefore contemplated. Therefore, a minimum or maximum amount of non-adhesive particles used may vary, depending upon a number of factors including, for example, the amount and type of adhesive particles used, the type of styling product, the remaining components and the like.

However, preferably, the minimum amount of non-adhesive particles in accordance with the present invention generally ranges from as little as about 0.03%, more preferably from about 0.04 weight percent based on the weight of the composition.

In addition, generally, the maximum amount of non-adhesive particles will not exceed about 2%, preferably not exceed about 0.3%, more preferably not exceed about 0.2%, weight percent based on the weight of the composition.

These are measurements by weight based on the total weight of the hair styling composition. Note that if a two-part composition is used such as often occurs with a bleaching system or a peroxide containing activator stored separately, the weight reported is not based on the total weight, but on the weight in whichever component the non-adhesive particles generally reside prior to mixing.

Alternatively, the amount of non-adhesive particles used in accordance with the present invention may be based on a ratio to the amount of adhesive particles used, rather than an absolute weight basis based on the weight of the total formulation. In an embodiment, the adhesive particles are provided in an amount that is equal to or greater than the amount of the non-adhesive particles. In another embodiment, the ratio of adhesive to non-adhesive particles is 1:1 to 250:1. In another embodiment, the ratio is from about 1:1 to about 100:1. In still another embodiment, the ratio is from about 2:1 to about 50:1 and in yet another embodiment from about 5:1 to about 45:1, and in still another embodiment, from about 10:1 to about 25:1. The amount of adhesive particles useful in accordance with the present invention will be discussed elsewhere herein.

Polymeric non-adhesive particles

The polymeric non-adhesive particles in accordance with the present invention are made from polymer materials. In accordance with the present invention, the terms "polymer" and "polymeric material" as used in connection with these particles, are synonymous and embraces both homopolymers and copolymers. Copolymers may be any type of copolymer including block copolymers. Any polymer may be used in accordance with the present invention as long as, when mixed with adhesive particles as described herein, it substantially maintains reshapable effect and/or imparts or enhances at least one desirable property such, without limitation, less tacky, less sticky, less greasy, silkier, smoother feel or appearance.

The selection of polymers for the non-adhesive particles may be influenced by a number of factors. For example, crosslinked polyacrylic esters having an average particle size of between about 8 and about 15 microns produced promising results in hair styling compositions in a hair styling gel format. Interestingly, the same material did not produce acceptable results in a mousse format. Materials that can be used include ACX-8066 and ACX-1502C available from Sekisui Plastics Co., Ltd., Functional Materials Division, 2-7-1 Nishi-Shinjuku, Shinjuku-ku, Tokyo 163-0727 Japan.

The above also demonstrates one aspect of the invention; namely the non-adhesive particles may be based on a single polymer species or single type of polymer particle. In addition to crosslinked polyacrylic esters, non-adhesive particles made of any single polymer species (homopolymer or copolymer), which may be useful in accordance with the present invention including, but not limited to polyamide-based resins, polyolefin-based resins, cellulose-based resins, a silicon-based resins, and fluorine-based resins. Successful polymers in accordance with the present invention will generally be cross-linked and will have a glass transition temperature of at least about 50°C, preferably about 100°C.

In another embodiment in accordance with the present invention, a physical mixture of non-adhesive particles of different materials is used. These will generally be cross-linked polymers having a glass transition temperature of at least about 50°C, preferably about 100°C. Indeed, it has been surprisingly found that by using mixtures of particles of different polymers, it is possible to provide an effective non-adhesive particle blend where the use of the individual component particles would not be effective or as effective. For example, it has been discovered that particles that otherwise fall within the criteria for non-adhesive particles made from a single material were found not to be effective when mixed with adhesive particles (Gel/Tac 205D having a particle size of 20 microns). Specifically, when non-adhesive particles of cross-linked polymethyl methacrylate were mixed with adhesive particles Gel/Tac 205D in a mousse format, the mixture did not provide acceptable properties. Similarly, non-adhesive particles of cross-linked polystyrene were

found to not be effective when mixed with adhesive particles Gel/Tac 205D in a mousse format. However, when a mixture was prepared using equal amounts of cross-linked polystyrene and cross-linked polymethyl methacrylate, quite surprisingly, acceptable properties were realized in the same mousse format.

Thus, the at least one non-adhesive particle of the present invention includes particles of any polymeric material, which when blended with particles of at least one other polymeric material, produce acceptable results in accordance with the present invention. This includes not only the polymers identified above for non-adhesive particles made from a single material, but also polymers, such as polystyrene and polymethyl methacrylate, which were found to have insufficient benefits, in particular formulation and format, when used alone. Therefore, polymers useful in accordance with this aspect of the present invention are considerably broader than the identification of polymers used above for at least one non-adhesive particle that is constituted of a single polymeric material. These polymers may therefore include (meth) acrylate-based resins, styrene resins, polyamide-based resins, polyolefin-based resins, cellulose-based resins, silicon-based resins, and fluorine-based resins.

Examples of the monomers that form the styrene-based resins are alkyl styrenes such as methylstyrene, dimethylstyrene, trimethylstyrene, ethylstyrene, diethylstyrene, triethylstyrene, propylstyrene, butylstyrene, hexylstyrene, heptylstyrene, octylstyrene, or the like; a halogenated styrene such as fluorostyrene, chlorostyrene, bromostyrene, dibromostyrene, iodostyrene, chloromethylstyrene, or the like; nitrostyrene; acetylstyrene; methoxystyrene; and the like.

Examples of monomers that form the (meth)acrylate-based resins are methyl (meth)acrylate, ethyl (meth) acrylate, propyl (meth)acrylate, butyl (meth)acrylate, 2-ethylhexyl (meth)acrylate, lauryl (meth)acrylate, stearyl (meth) acrylate, cyclohexyl (meth)acrylate, 2-hydroxyethyl (meth)acrylate, 2-propyl (meth)acrylate, chloro-2-hydroxyethyl (meth)acrylate, diethylene-glycol mono(meth)acrylate, methoxy ethyl (meth)acrylate, glycidyl (meth)acrylate, dicyclopentanyl (meth)acrylate, isobronol (meth)acrylate, and the like. In one preferred embodiment, one of the polymers used in the particle blend will be appreciably less hydrophobic than the other.

When a styrene-based resin and a (meth)acrylate-based resin are used as blended particles, it is preferred that homopolymers of the monomers described above are used. However, copolymers formed from two or more kinds of monomers described above may be employed if they satisfy the refractive index disclosed in the Japanese application publication no. JP-2001-294513.

When prepared in a blend, the amount of one polymer to the other (assuming a two polymer system and as many polymers may be used as desired) is any effective amount to produce acceptable results as that term was previously defined. However, generally, the ratio of one polymer to the other polymer will range from between about 2.5:1 to about 1:2.5, more preferably about 2:1 to about 1:2. Even more preferably the ratio will be between about 1.5:1 to about 1:1.5 and most preferably about 1:1.

In another preferred embodiment in accordance with the present invention, a non-adhesive particle with a core/shell structure is used. "Core/shell" particles are particles having a core of one polymer material and a shell made of another. The particle size recitations previously described for non-adhesive particles apply to the total size of both the core and the shell of these particles. The thickness of the shell is preferably, based on spherical particles of mean radius of 4 microns, 0.13-0.45 microns. A proportionate thickness will apply to larger or smaller particles.

Any of the materials useful for forming a mixture of non-adhesive particle materials may be used to produce core/shell materials. For example, core/shell particles that are useful in accordance with the present invention may be produced using a core of polystyrene and a shell of polymethyl methacrylate. Both are cross-linked and have glass transition temperatures as described before.

Regarding the particles with a core/shell structure, the preferred material for the core is a relatively hydrophobic and the shell is relatively hydrophilic. A preferred core is made of a styrene resin, and the preferred material is a (meth) acrylate-based resin. These polymers with core/shell structure are disclosed in the Japanese application publication no. JP-2001-294513, the disclosure of which relating to polymers with core/shell structure and how they are produced is incorporated herein by reference. These core/shell materials are sold, for example, under the name Techpolymer by Sekisui Plastics Co. Ltd., Japan. Core/shell polymers can be used alone, in combination with other core/shell particles, or with single or blends of particles as previously disclosed.

Note that by "core/shell" particles, the term core/shell actually covers a number of possible configurations or structural relationships between two or more polymers. Representative examples of various structures which fall in to the term "core/shell" are found in Figure 1. They include, without limitation, core/shell (truly a core surrounded uniformly by a shell of the second material), inversed core/shell, raspberry, sandwich, champignon, salami, interpenetrated and moon-like. These are, of course, nonlimiting examples of these relationships.

INORGANIC NON-ADHESIVE PARTICLES

The inorganic non-adhesive particle can be made of any nonpolymeric material such as, without limitation, cosmetically acceptable pigments and mineral powders. As used herein, a "non-adhesive" particle is a particle that is not coated with an adhesive as described in U.S. Patent No. 6,548,051 and U.S. Patent Application Publication No.

2002/0059941. More specifically, a non-adhesive particle of the invention is a discrete substrate that is not an adhesive and is not coated with an adhesive coating in whole or in part, and does not comprise an adhesive as a core or shell material when in a core/shell structure.

It is not necessary that the term particle embrace particles of a particular shape or geometry. However, the inorganic non-adhesive particles in accordance with the present invention will generally be roughly spherical, having an aspect ratio of from about 1.0 to about 1.2. Particle size generally ranges from between about 2 to about 80 microns, more preferably from about 8 to about 70 microns and most preferably about 15 to about 60 microns. Particle size in accordance with the present invention means mean particle size by volume as measured by a laser beam diffraction MALVERN 2000. The inorganic non-adhesive particles in accordance with the present invention are made from inorganic materials. Any inorganic particles may be used in accordance with the present invention as long as, when mixed with adhesive particles as described herein, it provides an acceptable result in reshapable effect and/or imparts or enhances at least one desirable cosmetic property such, without limitation, less tacky, less sticky, less greasy, silkier, smoother feel or appearance.

The selection of inorganic materials for the inorganic non-adhesive particles may be influenced by a number of factors. For example, Biron® liquid Silver and Xirona® Nordic Sunset (silica, titanium dioxide and tin oxide), each having an average particle size of between about 15 and about 20 microns, produced promising results in hair styling compositions in a hair styling mousse format. Interestingly, the same materials did not produce acceptable results in a gel format. Materials that can be used include Omyapur® 35 (OMYA SAS, 35 quai André Citroën 75725 Paris Cedex 15, France), Luxsil® (Rossow Cosmétiques, 6/8 Place Jean Zay-BP16, 92301 Levallois Perret Cedex, France); Biron® Liquid Silver, Xirona® Nordic Sunset and Timiron® Diamond Cluster MP149 (Rona - Cosmetic Business Unit, EMD Chemicals Inc., 7 Skyline Drive, Hawthorne, New York 10532, USA), and Flamenco Satin Gold (Engelhard, 101 Wood Avenue, Iselin, NJ 08830-0770, USA).

ADHESIVE PARTICLE AND ENCAPSULATED PARTICLE

The description of the adhesive particle and encapsulated particle, as used herein, is disclosed in U.S. Patent No. 6,548,051 and U.S. Patent Application Publication No. 2002/0059941, the disclosure of which is incorporated by reference.

The term "adhesive particle" encompasses encapsulated, and non-encapsulated adhesive particles, as well as a mixture of encapsulated and non-encapsulated adhesive particles unless the context dictates otherwise.

In one embodiment, an adhesive particle is a coated discrete substrate, which comprises at least one substrate coated with an at least partial coating that comprises at least one adhesive, effective in providing a composition with a holding and/or reshapable effect. In one embodiment, the at least one adhesive particle remains tacky or sticky following evaporation of the carrier. It is not necessary for the at least one adhesive particle to be spherical or any particular shape, and there is no requirement that the at least one adhesive particle be solid or have a specific hardness. More specifically, an adhesive particle of the invention is either (a) a discrete substrate that is not an adhesive and is at least partially coated with a coating comprising at least one adhesive or (b) a discrete substrate that is formed from or comprises, in whole or in part, at least one adhesive and is also at least partially coated with a coating comprising at least one adhesive. In one embodiment, the at least one substrate and the at least partial coating comprise the same adhesive. In another embodiment, the at least one substrate and the at least partial coating do not comprise the same adhesive.

With respect to the at least partial coating comprising at least one adhesive, the thickness of the coat is not critical; however, the at least one adhesive particle is most effective if a substantial portion of the surface area of the particle is covered. An acceptable partial coating is one where the coating is present to a degree such that there is an effective amount of the at least one adhesive to adhere to keratinous fibers. As used herein, to be coated, whether partially or fully, means that at least part of the surface of the adhesive particle has adhesive properties due to the coating. One of ordinary skill in the art would recognize that an at least partial coating may comprise various layers and may comprise constituents other than the at least one adhesive. An at least partial coating comprising at least one adhesive may be achieved, for example, by the addition of at least one adhesive to at least part of the surface or the treatment of at least part of the surface of the at least one substrate by physical or chemical means, such that at least part of the surface is rendered adhesive.

As for the at least one substrate, the materials that could be used in the practice of the invention include adhesives and any particle, e.g., inert particle, that does not substantially decrease desired adhesive and/or reshapable properties of the at least partial coating comprising at least one adhesive. Representative inert substrates include, but are not limited to, polymeric materials, such as polyethylene, polypropylene, and polyacrylates; metal alloys; metal oxides; ceramics; and glasses. In one embodiment, each adhesive particle comprises a mix of ceramic and glass substrates at least partially coated by an adhesive. In another embodiment, both the at least one substrate and the at least one adhesive of the at least partial coating are the same adhesive. In yet another embodiment, the at least one substrate and the at least one adhesive of the at least partial coating are not the same adhesive. In yet a further

embodiment, the at least one substrate and the at least partial coating are not the same, e.g., when both the at least one substrate and the at least partial coating each comprise the same adhesive, then either the at least one substrate or the at least partial coating, or both, must further comprise a suitable material not present in the other.

As used herein, an "encapsulated adhesive" is a particle comprising at least one adhesive encapsulated by at least one encapsulating material, wherein the at least one adhesive is effective for holding and/or reshaping hair fibers when the at least one encapsulating material is ruptured. It is not necessary for the particle to be spherical or any particular shape, and there is no requirement that the encapsulating material be solid or have a specific hardness. To be "encapsulated" means that the at least one adhesive is surrounded by the at least one encapsulating material. The term "adhesive particle" is meant to encompass both encapsulated and nonencapsulated adhesive particles unless the context dictates otherwise.

As used herein, an "encapsulating material" is a non-adhesive, rupturable coating that is not degraded or destroyed by the optional cosmetically acceptable carrier. The absolute thickness of the material is not critical; however, it is varied according to its mechanical strength.

In one embodiment, for example, the at least one encapsulated adhesive is most effective when the encapsulating material is thick enough not to rupture when expelled by a dispenser, such as an aerosol can, but thin enough to rupture when the hair is brushed or combed. This does not mean that no portion of the at least one encapsulated adhesive may be ruptured when expelled from the dispenser. In another embodiment, the thickness of the encapsulating material is chosen such that a portion of the encapsulated adhesives ruptures when they are expelled to provide an initial hold for the hair. Others may rupture when the user attempts to modify or restore the hairstyle. One of ordinary skill in the art would be able to identify the thickness needed for a given encapsulating material to rupture under a desired degree of force, such as shear stress, or other forms of mechanical pressure.

The encapsulating materials that could be useful in the practice of the invention include materials that do not substantially decrease the adhesive and/or reshapable properties of the at least one adhesive. Representative materials include, but are not limited to, film-forming materials, such as polymers, including starch derivatives. A suitable example of starch derivatives is DRY FLO Plus by National Starch.

In one embodiment, useful examples of encapsulating material include the compounds of U.S. Patent No. 3,729,569, the disclosure of which is incorporated herein by reference. In one embodiment, for example, the at least one encapsulating material is in the form of microcapsules, characterized by the nature of their envelope, their dimensions, their thickness, or by the ratio R between the weight of the envelope and the total weight of the microcapsule, as explained in U.S. Patent No. 3,729,569. The at least one adhesive is encapsulated by the at least one encapsulating material via processes well known in the art, including, but not limited to spray drying, freeze-drying, and drying under a vacuum. In one embodiment, a liposome is formed. The at least one encapsulating material may be prepared according to conventional processes, including those in situ.

The size, shape, and/or total surface area of the at least one adhesive particle can vary widely. It is helpful but not required for the at least one adhesive particle to possess a surface such that the strands of hair can position themselves along the surface and can touch the at least one adhesive of the coating of the at least one adhesive particle when the encapsulating material is ruptured. While not wishing to be bound by theory, the at least one adhesive particle, and even the particles of irregular shape, may offer enough surface area to allow for the hair or hair strands to be held together through a bridging by the at least one adhesive particle. For example, adhesive particles that have a long side and can position themselves with their long side parallel or substantially parallel to the hair, may bring about better holding and/or reshaping of the hair even after the hair has been already set or styled. Alternatively, the at least one encapsulated adhesive particle may bring about better holding and/or reshaping of the hair when the at least one encapsulating material is ruptured even after the hair has been already set or styled.

The at least one adhesive particle of the present invention may be of widely varied shapes such as spheres, spheroids, rods, platelets, flakes and irregularly shaped particles. The size of the at least one substrate and/or at least one adhesive particle is usually described in terms of its length and/or width. In the context of the present invention, "length" of at least one substrate and/or at least one adhesive particle is intended to mean the maximum distance possible to be measured by appropriate microscopy techniques, between two opposing points of the substrate and/or particle. The "width" of the at least one substrate and/or at least one adhesive particle is intended to mean the minimum distance possible to be measured by appropriate microscopy techniques, between two opposing points of the substrate and/or particle.

In one embodiment, the at least one substrate and/or at least one adhesive particle has a ratio of length to width of greater than or equal to about 1:1.

In another embodiment, the at least one adhesive particle has a mean length greater than 1 micron, such as from about 5 to about 1000 microns and such as from about 10 to about 100 microns. The mean length of the at least one adhesive particle may be measured by the following methods: optical microscopy, sieving, sedimentation, radiant diffusion, absorption, Coulter's principle, laser light diffraction, X-ray, and Sensing-zone method, which are described by Clyde Orr, "Size Measurement of Particles," in Kirk-Othmer Encyclopedia of Chemical Technology, vol. 21, pp.

106-131 (3rd ed. 1983) and by Terry Allen, "Analyse et Caracterisation, Technique de l'ingenieur" in Etudes de Structure-Granulometrie, vol.12, pp.1040-9 to 1040-26 (1996). In one embodiment, optical microscopy is used to determine the mean length of the at least one adhesive particle.

In one embodiment, the at least one adhesive particle useful in the practice of the invention may be chosen by measuring the maximum tensile force, $F_{max}$, required while separating two surfaces. An adhesive particle with an $F_{max}$ that is effective for holding and/or reshaping hair may be useful in the practice of the invention. Depending on the application envisaged and the formulation being designed, the desirable value for $F_{max}$ may vary. In some embodiments, adhesive particles with an $F_{max}$ of greater than about 0.5 Newton (N), greater than about 1 N, or greater than about 4 N may be useful in the practice of the invention. One of ordinary skill in the art can determine the $F_{max}$ of the at least one adhesive particle by, for example, determining the maximum force of traction, measured with an extensometer of the LLOYD model LR5K type, needed to detach two surfaces.

In one embodiment, two 38 $mm^2$ surfaces, A and B, which are solid, rigid, inert, and non-absorbing, are mounted on movable mounts, facing each other. The surfaces may be movable either toward or away from each other, or one may move surface A independently from surface B or vice versa. Prior to insertion into the extensometer, surface A is coated with the at least one adhesive particle and possibly at least one encapsulated adhesive previously dispersed at a concentration of 10% in a solvent chosen from aqueous, hydroalcoholic, and alcoholic solvents at a level of 1 $mg/mm^2$. The level surface is dried for 24 hours at 22°C at a relative humidity of 50%. Once inserted in the extensometer, surface A is subjected for 20 seconds to a compression force of 3 N against surface B (which breaks the at least one encapsulating material), and then subjected for 30 seconds to tensile force at a rate of 20 mm/minute. The amount of force, $F_{max}$, needed to obtain initial separation is then noted. A mean $F_{max}$ is determined by carrying out the procedure with six pairs of surface A and surface B.

In another embodiment, the at least one adhesive particle useful in the practice of the invention may be chosen based on the energy of separation, $E_s$, the energy supplied by the extensometer to separate two surfaces. One of skill in the art may determine the $E_s$ of the at least one adhesive particle using the LLOYD model LR5K type extensometer and other experimental procedure described in the preceding paragraphs.

In one embodiment, two 38 $mm^2$ surfaces, C and D, which are solid, rigid, inert, and non-absorbing, are mounted on movable mounts, facing each other. Only surface C is coated, as above, with the at least one adhesive particle dispersed in a solvent. The $E_s$ is measured with the LLOYD extensometer and may be calculated from the following formula:

$$\int_{Xsl+0.05}^{Xs\,2} F(x)\,dx \qquad \text{(I)}$$

where F(x) is the force required to produce a displacement (x); $X_{s1}$ is the displacement, expressed in millimeters, produced at the maximum tensile force; and $X_{s2}$ is the displacement, expressed in millimeters, produced by the tensile force, which permits the total separation of the two surfaces, C and D.

In one embodiment, the at least one adhesive particle useful in the practice of the invention has an $E_s$ of less than 300 μJ when placed in contact with a glass surface. For example, the adhesives of WO 98/38969, the disclosure of which related to such adhesives is incorporated herein by reference, in particular the anionic and nonionic adhesives, may be useful in the manufacture of the at least one adhesive particle.

The adhesives that may be useful in formulating the at least one adhesive of the at least partial coating and/or the at least one substrate of the invention are not limited to polymeric adhesives. Useful adhesives may be nonsoluble in the optional cosmetically acceptable carrier. In particular, by "adhesive" is meant that when applied as a solution to a surface, e.g., the hair or skin, and dried the adhesive forms films or welds. Such a film or weld will have adhesive and cohesive strength, as is understood by those skilled in the art. Useful examples include the adhesives of WO 98/48770, the disclosure of which related to such adhesives is incorporated herein by reference.

In one embodiment, the adhesive that may be useful in formulating the at least one adhesive of the at least partial coating and/or the at least one substrate of the invention is chosen from polymeric adhesives, for example fixing polymers, such as anionic, cationic, amphoteric (such as zwitterionic), and nonionic fixing polymers and combinations thereof. As used herein, the term "polymer" refers to homopolymers and copolymers, the copolymers being derived from more than one type of monomer, such as from two, three, four, or more different monomer types.

The cationic fixing polymers comprise cationic moieties or moieties that are convertible to cationic moieties. Suitable examples of cationic fixing polymers, which can be used according to the present invention, are those that may be chosen from polymers comprising at least one group chosen from primary amine groups, secondary amine groups, tertiary amine groups, and quaternary amine groups, wherein the at least one group forms part of the polymer chain or

is linked directly to it, having a weight average molecular weight ranging from about 500 to about 5,000,000, such as from about 100 to about 3,000,000.

Among these polymers, mention may be made more particularly of the following cationic fixing polymers:

(1) homopolymers and copolymers derived from monomers chosen from (meth)acrylic esters and (meth)acrylic amides comprising units of at least one of the following formulae:

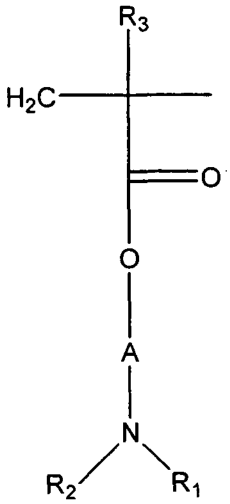

(A)

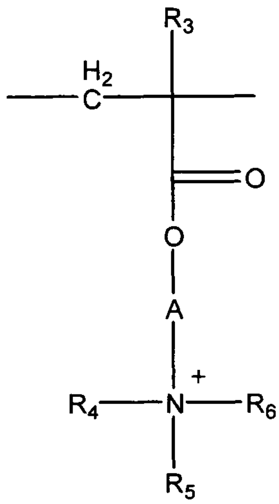

(B)

or

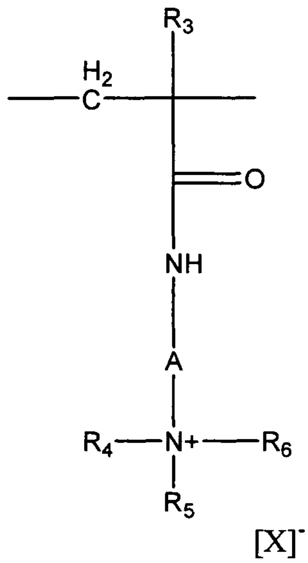

(C)

in which each $R_3$ is independently chosen from hydrogen and $CH_3$ groups; each A is independently chosen from linear and branched alkyl groups comprising 1 to 6 carbon atoms and hydroxyalkyl groups comprising 1 to 4 carbon atoms; each $R_4$, $R_5$, and $R_6$ is independently chosen from alkyl groups comprising 1 to 18 carbon atoms and benzyl groups; each $R_1$ and $R_2$ is independently chosen from hydrogen and alkyl groups comprising 1 to 6 carbon atoms; and each $X^-$ is independently chosen from methyl sulfate anions and halide anions, such as chloride or bromide anions.

In one embodiment, the copolymers of family (1) further comprise at least one unit derived from monomers chosen from (meth)acrylamides, diacetone (meth)acrylamides, (meth)acrylamides substituted on the nitrogen by a group chosen from lower alkyls, (meth)acrylic acids, esters of (meth)acrylic acids, vinyllactams such as vinylpyrrolidone and vinyl-caprolactam, and vinyl esters.

Thus, mention may be made, among these cationic copolymers of the family (1), of: copolymers of acrylamide and of dimethylaminoethyl methacrylate quaternized with dimethyl sulfate or with a dimethyl halide, such as that sold under the name Hercofloc by the company Hercules; copolymers of acrylamide and of methacryloyloxyethyltrimethylammonium chloride which are disclosed, for example, in EP-A-080,976, the disclosure of which relating to cationic polymers is incorporated herein by reference, and sold, for example, under the name Bina Quat P 100 by the company Ciba-Geigy; copolymers of acrylamide and of methacryloyloxyethyltrimethylammonium methosulfate, such as that sold under the name Reten by the company Hercules; optionally quaternized vinylpyrrolidone/dialkyl-aminoalkyl (meth)acrylate copolymers, which are disclosed, for example, in French Patents 2,077,143 and 2,393,573, the disclosures of which relating to cationic polymers are incorporated herein by reference, and sold, for example, under the name "Gafquat" by the company ISP, such as, for example, "Gafquat 734" or "Gafquat 755", or else the products named "Copolymer 845, 958 and 937"; dimethylaminoethyl methacrylate/vinylcaprolactam/vinylpyrrolidone terpolymers, such as the product sold under the name Gaffix VC 713 by the company ISP, and the quaternized vinylpyrrolidone/dimethylaminopropylmethacrylamide copolymer, such as the product sold under the name "Gafquat HS 100" by the company ISP;

(2) the quaternized polysaccharides, disclosed more particularly in U.S. Patent Nos. 3,589,578 and 4,031,307, the disclosures of which relating to quaternized polysaccharides polymers are incorporated herein by reference, such as guar gums comprising cationic trialkylammonium cationic groups. Such products are sold in particular under the trade names Jaguar C 13 S, Jaguar C 15, and Jaguar C 17 by the company Meyhall.

(3) quaternized copolymers of vinylpyrrolidone and of vinylimidazole, such as the products sold by BASF under the name Luviquat TFC.

(4) chitosans or their salts. The salts, which can be used, are in particular chitosan acetate, lactate, glutamate, gluconate, or pyrrolidone-carboxylate.

Mention may be made, among these compounds, of the chitosan having a degree of deacetylation of 90.5% by weight sold under the name Kytan Crude Standard by the company Aber Technologies and the chitosan pyrrolidone-carboxylate sold under the name Kytamer PC by the company Amerchol.

(5) Cationic cellulose derivatives, such as the copolymers of cellulose and the cellulose derivatives grafted with a water-soluble quaternary ammonium monomer and disclosed in particular in U.S. Patent No. 4,131,576, the disclosure of which relating to cationic cellulose derivatives is incorporated herein by reference. Examples include hydroxyalkyl celluloses, for example hydroxymethyl, hydroxyethyl, and hydroxypropyl celluloses grafted in particular with a methacryloylethyltrimethylammonium, methacrylamidopropyltrimethylammonium, or diallyldimethylammonium salt.

The commercial products corresponding to this definition are more particularly the products sold under the name "Celquat L 200" and "Celquat H 100" by the company National Starch.

The anionic fixing polymers, which can be used according to the present invention, are polymers comprising groups derived from carboxylic, sulfonic, and/or phosphoric acid and having a weight average molecular weight ranging from about 500 to about 5,000,000.

(1) The carboxyl groups may be contributed by unsaturated mono- or dicarboxylic acid monomers such as those corresponding to the formula:

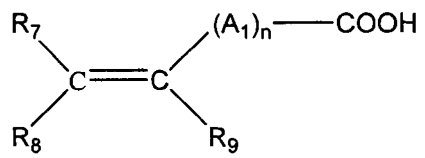

(II)

in which n is an integer ranging from 0 to 10; $A_1$ denotes a methylene group and when n is greater than 1, each $A_1$ is independently represented by $--LCH_2--$, where L is chosen from a valency bond and heteroatoms, such as oxygen and sulfur; $R_7$ is chosen from hydrogen, phenyl groups, and benzyl groups; $R_8$ is chosen from hydrogen, lower alkyl groups, and carboxyl groups; and $R_9$ is chosen from hydrogen, lower alkyl groups, $--CH_2--COOH$ groups, phenyl groups, and benzyl groups.

As defined herein, a lower alkyl group denotes a group having 1 to 4 carbon atoms, such as methyl and ethyl.

The anionic fixing polymers comprising carboxyl groups according to the invention may be chosen from:

A) Homopolymers and copolymers of (meth)acrylic acids or (meth)acrylic salts and in particular the products sold under the names Versicol E or K by the company Allied Colloid and Ultrahold by the company BASF, the copolymers of acrylic acid and of acrylamide sold in the form of their sodium salt under the names Reten 421, 423, or 425 by the company Hercules, and the sodium salts of polyhydroxycarboxylic acids.

B) Copolymers of (meth)acrylic acid with a monoethylenic monomer, such as ethylene, styrene, vinyl esters, and (meth)acrylic acid esters, optionally grafted onto a polyalkylene glycol, such as polyethylene glycol, and optionally crosslinked. Such polymers are disclosed in particular in French Patent 1,222,944 and German Application 2,330,956, the disclosures of which relating to such copolymers are incorporated herein by reference. The copolymers of this type comprising, in their chain, an optionally N-alkylated and/or hydroxyalkylated acrylamide unit, such as disclosed in particular in Luxembourg Patent Applications 75370 and 75371, the disclosures of which relating to such copolymers are incorporated herein by reference, or sold under the name Quadramer by the company American Cyanamid. Mention may also be made of copolymers of acrylic acid and of $C_1$ -$C_4$ alkyl methacrylate and terpolymers of vinylpyrrolidone, of acrylic acid, and of $C_1$ -$C_{20}$ alkyl methacrylate for example lauryl methacrylate, such as that sold by the company ISP under the name Acrylidone LM, and methacrylic acid/ethyl acrylate/tert-butyl acrylate terpolymers, such as the product sold under the name Luvimer 100 P by the company BASF.

C) copolymers derived from crotonic acid, such as those comprising, in their chain, vinyl acetate or propionate units and optionally other monomers, such as (meth)allyl esters, vinyl ether or vinyl ester of a linear or branched saturated carboxylic acid comprising a long hydrocarbon chain, such as those comprising at least 5 carbon atoms, it optionally being possible for these polymers to be grafted and crosslinked, or alternatively a vinyl, allyl, or methallyl ester of an α- or β-cyclic carboxylic acid. Such polymers are disclosed, inter alia, in French Patents 1,222,944, 1,580,545, 2,265,782, 2,265,781, 1,564,110, and 2,439,798, the disclosures of which relating to copolymers of crotonic acid are incorporated herein by reference. Commercial products coming within this class are the Resins 28-29-30, 26-13-14, and 28-13-10 sold by the company National Starch.

D) copolymers derived from $C_4$ -$C_8$ monounsaturated carboxylic acids or anhydrides chosen from: copolymers comprising units derived from (i) at least one monomer chosen from maleic, fumeric, and itaconic acids and anhydrides thereof and (ii) at least one monomer chosen from vinyl esters, vinyl ethers, vinyl halides, phenylvinyl derivatives, acrylic acids, and acrylic acid esters, the anhydride functional groups of these copolymers optionally being monoesterified or monoamidated. Such polymers are disclosed in particular in U.S. Patent Nos. 2,047,398, 2,723,248, and 2,102,112 and GB 839,805, the disclosures of which relating to such copolymers are incorporated

herein by reference, and in particular those sold under the names Gantrez AN or ES by the company ISP. copolymers comprising units derived from (i) at least one monomer chosen from maleic, citraconic, and itaconic anhydrides and (ii) at least one monomer chosen from (meth)allyl esters, optionally comprising in their chain at least one unit derived from groups chosen from (meth)acrylamide, α-olefin, (meth)acrylic ester, (meth)acrylic acid, and vinylpyrrolidone groups. The anhydride functional groups of these copolymers optionally are monoesterified or monoamidated.

These polymers are, for example, disclosed in French Patents 2,350,384 and 2,357,241 the disclosures of which relating to such copolymers are incorporated herein by reference.

E) polyacrylamides comprising carboxylate groups. (2) The anionic fixing polymers comprising sulfonic groups may be chosen from polymers comprising units, such as those derived from vinylsulfonic, styrenesulfonic, naphthalenesulfonic, and acrylamidoalkylsulfonic acids and their derivatives. These polymers may be chosen from: salts of polyvinylsulfonic acid having a weight average molecular weight that ranges from about 1000 to about 100,000, as well as the copolymers derived from at least one unsaturated comonomer, such as acrylic and methacrylic acids, their esters, acrylamides, their derivatives, vinyl ethers, and vinylpyrrolidone; salts of polystyrenesulfonic acid, the sodium salts having a weight average molecular weight ranging from about 100,000 to about 500,000, which are sold respectively under the names Flexan 500 and Flexan 130 by National Starch. These compounds are disclosed in Patent FR 2,198,719, the disclosure of which relating to salts of polystyrenesulfonic acid is incorporated herein by reference; salts of polyacrylamidesulfonic acids, including those mentioned in U.S. Patent No. 4,128,631, the disclosure of which relating to salts of polyacrylamidesulfonic acid is incorporated herein by reference, and more particularly the polyacrylamidoethylpropanesulfonic acid sold under the name Cosmedia Polymer HSP 1180 by Henkel.

In one embodiment, the anionic fixing polymers are chosen from acrylic acid copolymers, such as the acrylic acid/ethyl acrylate/N-tert-butylacrylamide terpolymer sold under the name Ultrahold Strong by the company BASF; copolymers derived from crotonic acid, such as the vinyl acetate/vinyl tert-butyl-benzoate/crotonic acid terpolymers and the crotonic acid/vinyl acetate/vinyl neododecanoate terpolymers sold under the name Resin 28-29-30 by the company National Starch; polymers derived from at least one monomer chosen from maleic, fumeric, and itaconic acids and anhydrides thereof and also from at least one monomer chosen from vinyl esters, vinyl ethers, vinyl halides, phenylvinyl derivatives, acrylic acid, and esters of acrylic acid, such as the monoesterified methyl vinyl ether/maleic anhydride copolymer sold under the name Gantrez ES 425 by the company ISP; copolymers of methacrylic acid and of methyl methacrylate sold under the name Eudragit L by the company Rohm Pharma; the copolymer of methacrylic acid and of ethyl acrylate sold under the name Luvimer MAEX or MAE by the company BASF; the vinyl acetate/crotonic acid copolymer sold under the name Luviset CA 66 by the company BASF; and the vinyl acetate/crotonic acid copolymer grafted by polyethylene glycol sold under the name Aristoflex A by the company BASF.

In another embodiment, the anionic fixing polymers are chosen from the monoesterified methyl vinyl ether/maleic anhydride copolymer sold under the name Gantrez ES 425 by the company ISP; the acrylic acid/ethyl acrylate/N-tert-butylacrylamide terpolymer sold under the name Ultrahold Strong by the company BASF; the copolymers of methacrylic acid and of methyl methacrylate sold under the name Eudragit L by the company Rohm Pharma; the vinyl acetate/vinyl tert-butylbenzoate/crotonic acid terpolymers and the crotonic acid/vinyl acetate/vinyl neododecanoate terpolymers sold under the name Resin 28-29-30 by the company National Starch; the copolymer of methacrylic acid and of ethyl acrylate sold under the name Luvimer MAEX or MAE by the company BASF; and the vinyl-pyrrolidone/acrylic acid/lauryl methacrylate terpolymer sold under the name Acrylidone LM by the company ISP.

The amphoteric fixing polymers, which can be used in accordance with the invention, may be chosen from polymers comprising X and Y units, distributed randomly in the polymer chain, where the X unit is chosen from units derived from at least one monomer comprising at least one basic function, in particular a basic nitrogen atom, and where the Y unit is chosen from units derived from at least one acidic monomer comprising at least one group chosen from carboxyl groups and sulfo groups, or else where each X and Y unit is independently chosen from groups derived from zwitterionic carboxybetaine and sulfobetaine monomers. In another embodiment, the amphoteric fixing polymers, which can be used in accordance with the invention, may be chosen from polymers comprising X and Y units, each X and Y unit is independently chosen from at least one cationic polymer chain comprising at least one group chosen from primary amine groups, secondary amine groups, tertiary amine groups, and quaternary amine groups, in which at least one of the amine groups comprises a group chosen from carboxyl and sulfo groups linked by way of a hydrocarbon group, or else the X and Y units, which may be different or identical, form part of a chain of at least one polymer comprising an α,β-dicarboxy ethylene unit, wherein at least one of the carboxyl groups has been reacted with a polyamine comprising at least one group chosen from primary and secondary amine groups.

In one embodiment, the amphoteric fixing polymers corresponding to the definition given above are chosen

from the following polymers: (1) polymers resulting from the copolymerization of a monomer derived from a vinyl compound carrying a carboxyl group, such as (meth)acrylic acids, maleic acids, and $\alpha$-chloracrylic acids, and of a basic monomer derived from a substituted vinyl compound comprising at least one basic atom, such as dialkylaminoalkyl (meth)acrylate and dialkylaminoalkyl (meth)acrylamide. Such compounds are disclosed in U.S. Patent No. 3,836,537, the disclosure of which relating to amphoteric polymers is incorporated herein by reference.

(2) polymers comprising units derived from:

a) at least one monomer chosen from (meth)acrylamides substituted on the nitrogen with an alkyl group,
b) at least one acidic comonomer comprising at least one reactive carboxylic group, and
c) at least one basic comonomer, such as esters comprising at least one substituent chosen from primary, secondary, tertiary, and quaternary amine substituents of (meth)acrylic acids, and the product of quaternization of dimethylaminoethyl methacrylate with dimethyl or diethyl sulfate.

The at least one N-substituted (meth)acrylamide monomer recited in (a) is more particularly chosen from N-substituted (meth)acrylamides, wherein the alkyl groups comprise from 2 to 12 carbon atoms, such as N-ethylacrylamide, N-tert-butylacrylamide, N-tert-octylacrylamide, N-octylacrylamide, N-decylacrylamide, N-dodecylacrylamide, and the corresponding methacrylamides.

The at least one acidic comonomer recited in (b) is more particularly chosen from (meth)acrylic acids, crotonic acids, itaconic acids, maleic acids, fumeric acids, $C_1$ -$C_4$ alkyl monoesters of maleic acid, $C_1$ -$C_4$ alkyl monoesters of fumeric acid, $C_1$ -$C_4$ alkyl monoesters of maleic anhydride, and $C_1$ -$C_4$ alkyl monoesters of fumeric anhydride.

The at least one basic comonomer recited in (c) is more particularly chosen from aminoethyl, butylaminoethyl, N,N'-dimethylaminoethyl, and N-tert-butylaminoethyl methacrylates.

In one embodiment, the amphoteric fixing polymer is chosen from the copolymers for which the CTFA name (4th Ed., 1991) is octylacrylamide/acrylates/butylaminoethyl methacrylate copolymer, such as the products sold under the name Amphomer or Lovocryl 47 by the company National Starch.

(3) crosslinked and alkylated polyamino amides partially or totally derived from polyamino amides of general formula:

$$\left[ CO-R_{10}-CO-Z \right] \qquad (III)$$

in which $R_{10}$ represents a divalent group derived either from a saturated dicarboxylic acid, from an aliphatic mono- or dicarboxylic acid comprising an ethylenic double bond, from an ester of a lower alkanol having 1 to 6 carbon atoms of these acids, or from a group derived from the addition of any one of the said acids with a bisprimary or bissecondary amine; and Z denotes a group of a bisprimary, mono- or bissecondary polyalkylenepolyamine and, for example, represents:

a) in the proportions of from about 60 mol% to 100 mol%, the group:

$$-NH\left[(CH_2)_x-NH\right]_p \qquad (IV)$$

where x=2 and p=2 or 3, or else x=3 and p=2 and where this group derives from diethylenetriamine, triethylenetetraamine, or dipropylenetriamine;

b) in the proportions of from 0 mol % to about 40 mol %, the above group (IV), in which x=2 and p=1 and which derives from ethylenediamine, or the group derived from piperazine:

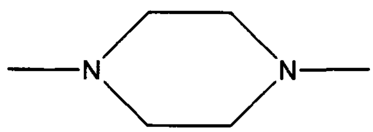

c) in the proportions of from 0 mol % to about 20 mol %, the -NH- $(CH_2)_6$ -NH- group derived from hexamethylenediamine, these polyamino amines being crosslinked by addition of a difunctional crosslinking agent chosen from epihalohydrines, diepoxides, dianhydrides and bis-unsaturated derivatives, using from about 0.025 mol to about 0.35 mol of crosslinking agent per amine group of the polyamino amide and alkylated by the action of acrylic acid, chloroacetic acid or an alkane sultone, or salts thereof.

In one embodiment, the saturated carboxylic acids are chosen from acids having 6 to 10 carbon atoms, such

as adipic acid, 2,2,4-trimethyladipic acid, 2,4,4-trimethyladipic acid, terephthalic acid, and acids comprising an ethylenic double bond such as, for example, acrylic acid, methacrylic acid and itaconic acid.
In one embodiment, the alkane sultones used in the alkylation are chosen from propane sultone and butane sultone and the salts of the alkylating agents are chosen from sodium and potassium salts.

(4) polymers comprising zwitterionic units of formula:

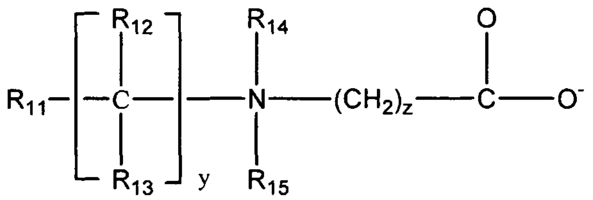

(V)

in which $R_{11}$ is chosen from polymerizable unsaturated groups such as a (meth)acrylate and (meth)acrylamide groups; y and z are independently chosen from integers ranging from 1 to 3; $R_{12}$ and $R_{13}$ are independently chosen from hydrogen, methyl groups, ethyl groups, and propyl groups; $R_{14}$ and $R_{15}$ are independently chosen from hydrogen and alkyl groups, wherein the sum of the carbon atoms in $R_{14}$ and $R_{15}$ is less than or equal to 10.
The polymers comprising such units may further comprise units derived from non-zwitterionic monomers, such as dimethylaminoethyl (meth)acrylate, diethylaminoethyl (meth)acrylate, alkyl (meth)acrylates, (meth)acrylamides, and vinyl acetates.
Mention may be made, by way of example, of the methyl methacrylate/ methyl dimethylcarboxymethylammonioethyl methacrylate copolymer, such as the product sold under the name Diaformer Z301 by the company Sandoz.
(5) polymers derived from chitosan comprising monomer units corresponding to the following formulae:

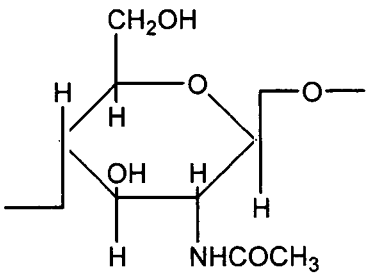

(D)

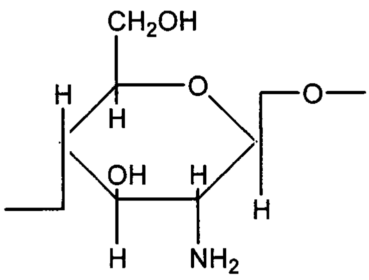

(E)

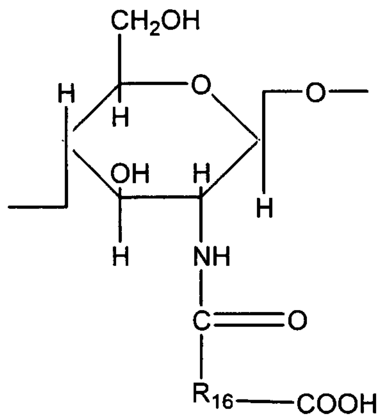

(E)

the unit D being present in proportions ranging from 0% to about 30%, the unit E in proportions ranging from about 5% to about 50% and the unit F in proportions ranging from about 30% to about 90%, it being understood that, in this unit F, $R_{16}$ represents a group of formula:

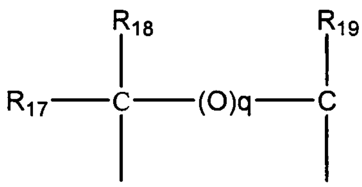

in which, if q=O, $R_{17}$, $R_{18}$, and $R_{19}$, which are identical or different, are chosen from hydrogen, methyl groups, hydroxyl groups, acetoxy groups, amino residues, monoalkylamine residues, and dialkylamine residues, optionally interrupted by one or more nitrogen atoms and/or optionally substituted by one or more amine, hydroxyl, carboxy, alkylthio, or sulfo groups, and alkylthio residues in which the alkyl group carries an amino residue, at least one of $R_{17}$, $R_{18}$, and $R_{19}$ being, in this case, hydrogen; or, if q=1, $R_{17}$, $R_{18}$, and $R_{19}$ each represent hydrogen, and the salts formed by these compounds with bases or acids.

(6) Polymers derived from the N-carboxyalkylation of chitosan, such as the N-(carboxymethyl)chitosan or the N-(carboxybutyl)chitosan sold under the name "Evalsan" by the company Jan Dekker.

(7) Polymers corresponding to the general formula (VI), for example disclosed in French Patent 1,400,366, the disclosure of which relating to amphoteric polymers is incorporated herein by reference:

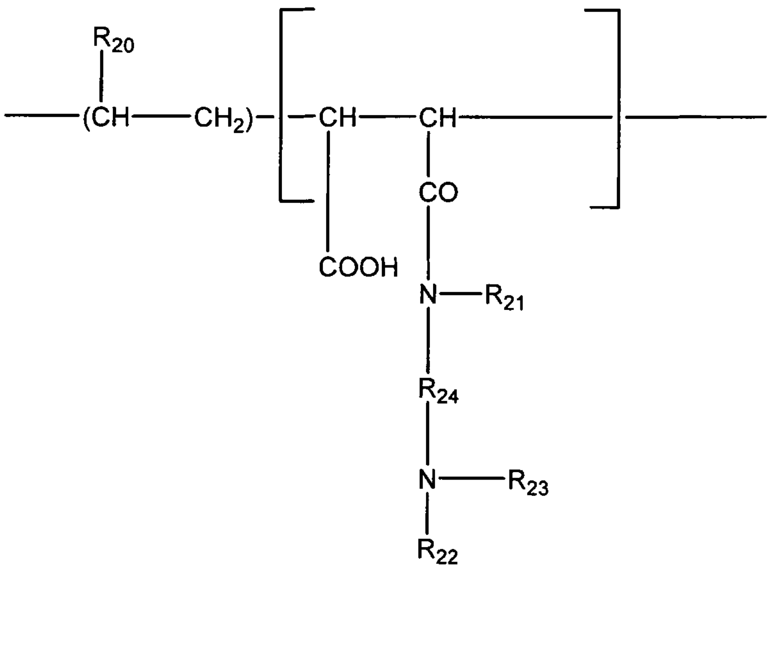

(VI)

in which $R_{20}$ is chosen from hydrogen, $CH_3O$, $CH_3CH_2O$, and phenyl groups; $R_{21}$ is chosen from hydrogen and lower alkyl groups such as methyl or ethyl; $R_{22}$ is chosen from hydrogen and lower alkyl groups such as methyl or ethyl; and $R_{23}$ is chosen from lower alkyl groups such as methyl or ethyl and groups corresponding to the formula: $-R_{24}-N(R_{22})_2$, where $R_{24}$ is chosen from $-CH_2-CH_2-$, $-CH_2-CH_2-CH_2-$, and $-CH_2-CH(CH_3)-$ groups and $R_{22}$ is the same as above, and the higher homologues of these groups comprising up to 6 carbon atoms.

(8) Amphoteric fixing polymers of the -D-X-D-X- type chosen from:

a) polymers obtained by reaction of chloracetic acid or sodium chloracetate with compounds comprising at least one unit of formula:

-D-X-D-X-D- (VII)

where D denotes a group

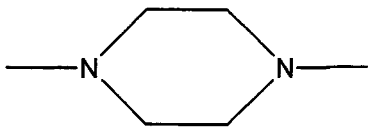

and X denotes the symbol E or E', E and E', which are identical or different, denote a divalent group chosen from straight- and branched-chain alkylene groups comprising up to 7 carbon atoms in the main chain, which is unsubstituted or substituted by hydroxyl groups and which can additionally comprise oxygen, nitrogen, or sulfur atoms or 1 to 3 aromatic and/or heterocyclic rings; the oxygen, nitrogen, and sulfur atoms being present in the form of ether, thioether, sulfoxide, sulfone, sulfonium, alkylamine or alkenylamine groups, benzylamine, amine oxide, quaternary ammonium, amide, imide, alcohol, ester, and/or urethane groups.

b) Polymers of formula:

-D-X-D-X- (VII')

in which D denotes a group

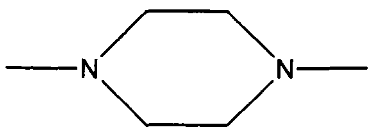

and X denotes the symbol E or E' and E' at least once, where E has the meaning indicated above and E' is a divalent group chosen from straight- and branched-chain alkylene groups having up to 7 carbon atoms in the main chain, which is substituted or unsubstituted by one or more hydroxyl groups and which comprises one or more nitrogen atoms, the nitrogen atom being substituted by an alkyl chain optionally interrupted by an oxygen atom and necessarily comprising one or more carboxyl functional groups or one or more hydroxyl functional groups and wherein the polymer of formula VII' is betainized by reaction with chloracetic acid or sodium chloracetate.

(9) ($C_1$-$C_5$)alkyl vinyl ether/maleic anhydride copolymers, which are partially modified by semiamidation with an N,N-dialkylaminoalkylamine, such as N,N-dimethylaminopropylamine, or by semiesterification with an N,N-dialkanolamine. These copolymers can also comprise other vinyl comonomers, such as vinylcaprolactam.

In one embodiment, the amphoteric fixing polymers according to the invention are chosen from family (3), such as the copolymers with the CTFA name (4th Ed. 1991) of octylacrylamide/acrylates/butylaminoethyl methacrylate copolymer, such as the products sold under the names Amphomer, Amphomer LV 71, or Lovocryl 47 by the company National Starch, and family (4), such as the copolymer of methyl methacrylate/dimethyl carboxymethylammonio methyl ethylmethacrylate, sold, for example, under the name Diaformer Z301 by the company Sandoz.

The nonionic fixing polymers, which can be used according to the present invention, are chosen, for example, from: vinylpyrrolidone homopolymers; copolymers of vinylpyrrolidone and of vinyl acetate; polyalkyloxazolines, such as

the polyethyloxazolines sold by the company Dow Chemical under the names PEOX 50 000, PEOX 200 000 and PEOX 500 000; vinyl acetate homopolymers, such as the product sold under the name Appretan EM by the company Hoechst or the product sold under the name Rhodopas A 012 by the company Rhone-Poulenc; copolymers of vinyl acetate and of acrylic ester, such as the product sold under the name Rhodopas AD 310 by Rhone-Poulenc; copolymers of vinyl acetate and of ethylene, such as the product sold under the name Appretan TV by the company Hoechst; copolymers of vinyl acetate and of maleic ester, for example of dibutyl maleate, such as the product sold under the name Appretan MB Extra by the company Hoechst; copolymers of polyethylene and of maleic anhydride; alkyl acrylate homopolymers and alkyl methacrylate homopolymers, such as the product sold under the name Micropearl RQ 750 by the company Matsumoto or the product sold under the name Luhydran A 848 S by the company BASF; acrylic ester copolymers such as, for example, copolymers of alkyl (meth)acrylates, such as the products sold by the company Rohm & Haas under the names Primal AC-261 K and Eudragit NE 30 D, by the company BASF under the names Acronal 601, Luhydran LR 8833 or 8845, and by the company Hoechst under the names Appretan N 9213 or N 9212; copolymers of acrylonitrile and of a nonionic monomer chosen, for example, from butadiene and alkyl (meth)acrylates; mention may be made of the products sold under the names Nipol LX 531 B by the company Nippon Zeon or those sold under the name CJ 0601 B by the company Rohm & Haas; polyurethanes, such as the products sold under the names Acrysol RM 1020 or Acrysol RM 2020 by the company Rohm & Haas, and the products Uraflex XP 401 UZ and Uraflex XP 402 UZ by the company DSM Resins; copolymers of alkyl acrylate and of urethane, such as the product 8538-33 by the company National Starch; polyamides, such as the product Estapor LO 11 sold by the company Rhone-Poulenc.; nonionic guar gums that are chemically modified or unmodified.

The unmodified nonionic guar gums are, for example, the products sold under the name Vidogum GH 175 by the company Unipectine and under the name Jaguar C by the company Meyhall.

The modified nonionic guar gums, which may be used according to the invention, are, for example, modified with $C_1$ -$C_6$ hydroxyalkyl groups. Examples, which may be mentioned, are hydroxymethyl, hydroxyethyl, hydroxypropyl, and hydroxybutyl groups.

These guar gums are well known in the prior art and may be prepared, for example, by reacting corresponding alkene oxides such as, for example, propylene oxides with guar gum so as to obtain a guar gum modified with hydroxypropyl groups.

Such nonionic guar gums, optionally modified with hydroxyalkyl groups, are sold, for example, under the trade names Jaguar HP8, Jaguar HP60, Jaguar HP120, Jaguar DC 293 and Jaguar HP 105 by the company Meyhall and under the name Galactosol 4H4FD2 by the company Aqualon.

The alkyl groups in the nonionic polymers comprise from I to 6 carbon atoms, except where otherwise mentioned.

According to the invention, it is also possible to use fixing polymers of grafted silicone type comprising a polysiloxane portion and a portion comprising a non-silicone organic chain, one of the two portions constituting the main chain of the polymer and the other being grafted onto the said main chain. These polymers are disclosed, for example, in EP-A-0,412,704, EP-A-0,412,707, EP-A-0,640,105, WO 95/00578, EP-A-0,582,152, and WO 93/23009 and U.S. Patent Nos. 4,693,935, 4,728,571, and 4,972,037, the disclosures of which relating to grafted silicone type polymers are incorporated herein by reference. These polymers are, for example, anionic or nonionic.

Such polymers are, for example, copolymers which can be obtained by radical polymerization from the monomer mixture comprising:

a) about 50% to about 90% by weight of tert-butyl acrylate;
b) 0% to about 40% by weight of acrylic acid;
c) about 5% to about 40% by weight of silicone macromonomer of formula:

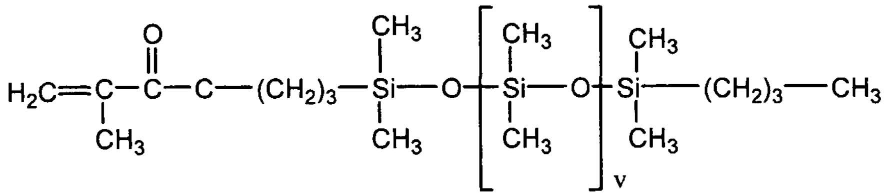

where v is a number ranging from 5 to 700; the percentages by weight being calculated with respect to the total weight of the monomers.

Other examples of grafted silicone polymers are, in particular, polydimethylsiloxanes (PDMSs) onto which are grafted, via a thiopropylene-type connecting chain, mixed polymer units of the poly (meth) acrylic acid type and of the

poly(alkyl (meth)acrylate) type and polydimethylsiloxanes (PDMSs) onto which are grafted, via a thiopropylene-type connecting chain, polymer units of the poly(isobutyl (meth)acrylate) type.

It is also possible to use, as fixing polymers, functionalized or non-functionalized and silicone-comprising or non-silicone-comprising polyurethanes.

Examples of useful polyurethanes include those disclosed in Patents EP 0,751,162, EP 0,637,600, FR 2,743,297, EP 0,648,485, EP 0,656,021, WO 94/03510, and EP 0,619,111, the disclosure of which relating to polyurethanes are incorporated herein by reference.

In a further embodiment, the fixing polymers may be used in solubilized form or may be in the form of dispersions of solid particles (latex or pseudo-latex).

In another embodiment, the at least one adhesive of the at least partial coating and/or the at least one substrate of the invention may be chosen from polymeric adhesives, such as (meth)acrylic copolymers comprising: (a) units derived from at least one monomer present at from about 0.1 to about 99% by weight, such as about 9 to about 99% by weight of the total weight of the polymer and (b) units derived from at least one co-monomer present at up to about 99.9% by weight, such as up to about 91% by weight.

The at least one monomer recited in (a) can generally be represented by formula:

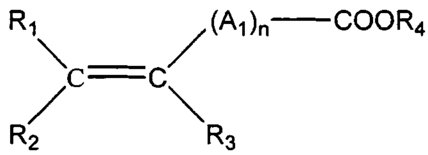

in which n is an integer ranging from 0 to 10; $A._1$ denotes a methylene group and when n is greater than 1, each $A_1$ is independently represented by - $LCH_2$ -, where L is chosen from a valency bond and heteroatoms, such as oxygen and sulfur; $R_1$ is chosen from hydrogen, phenyl groups, and benzyl groups; $R_2$ is chosen from hydrogen, lower alkyl groups, and carboxyl groups; and $R_3$ is chosen from hydrogen, lower alkyl groups, - $CH_2$ - COOH groups, phenyl groups, and benzyl groups; and $R_4$ is chosen from hydrogen, $C_1$ to $C_{18}$ alkyls, $C_2$ to $C_8$ alkoxyalkyls, $C_2$ to $C_8$ alkylthioalkyls, and $C_2$ to $C_8$ cyanoalkyls.

The at least one monomer recited in (a) may, for example, be chosen from acrylic acids, methacrylic acids, salts thereof, and derivatives thereof, such as acrylic and methacrylic esters, including methyl acrylate, ethyl acrylate, n-butyl acrylate, isobutyl acrylate, hexyl acrylate, octyl acrylate, 2-ethylhexyl acrylate, decyl acrylate, methoxyacrylate, ethoxyacrylate, methylthiomethyl acrylate, and cyanopropyl acrylate.

The at least one co-monomer recited in (b) may contain one or more terminal $CH_2$ =C groups, for instance, acrylic or methacrylic esters such as methyl methacrylate, ethyl methacrylate, n-butyl methacrylate, and methyl methacrylate; vinyl halides such as vinyl chlorides; vinyl or allyl esters such as vinyl acetate, vinyl butyrate, and vinyl chloroacetate; and aromatic vinyls such as styrene, vinyltoluene, chloromethylstyrene, and vinylnaphthalene.

In a further embodiment, these (meth)acrylic copolymers may further comprise (c) units derived from at least one vinylidene co-monomer containing at least one group chosen from carboxyl and hydroxyl groups present at from about 1 to about 10% by weight.

Among the at least one vinylidene co-monomer recited in (c) containing at least one hydroxyl group, one may use, for example, acrylate monomers with a terminal hydroxyl group, such as hydroxyethyl acrylate, hydroxyethyl methacrylate, hydroxypropyl acrylate or some other hydroxymethylated diacetone acrylamide derivatives such as N-methylol acrylamide, N-methylol maleamide, N-propanolacrylamide, N-methylol methacrylamide or N-methylol-p-vinyl benzamide. The at least one vinylidene co-monomer recited in (c) containing at least one carboxyl group may be chosen from, for instance, acrylic acid, methacrylic acid, itaconic acid, citraconic acid, crotonic acid, and maleic acid.

Examples of adhesive particles useful in the practice of the invention include the Gel-Tac 100 A, 100 B, 100 C, and 100K Microspheres (API Company), which have a diameter (mean length) of 35 microns, 45 microns, 25 microns, and 10 microns, respectively. The Gel-Tac Microspheres comprise a substrate coated with an acrylic polymer. Other adhesives that may be useful in manufacture of hair styling compositions include the polymers described in WO 98/53794, WO 98/48772, WO 98/48771, WO 97/15725, WO 99/00105, WO 93/23446, WO 96/32920, WO 98/51276, and WO 98/49213, and U.S. Patent Nos. 5,019,377, 4,963,348, and 5,565,193, the disclosures of which related to adhesives are incorporated herein by reference.

In yet another embodiment of the invention, the adhesive that may be useful in formulating the at least one adhesive of the at least partial coating and/or the at least one substrate of the at least one adhesive particle or the at least one encapsulated adhesive particle has a glass transition temperature (Tg) ranging from about -100°C. to about 15°C. The Tg of the adhesive is obtained following the application of the adhesive to a substrate and drying. The glass transition temperature is determined by the Differential Scanning Calorimetric method (DSC).

FORMULATIONS COMPRISING AT LEAST ONE NON-ADHESIVE PARTICLE AND AT LEAST ONE ADHESIVE PARTICLE

The at least one adhesive particle and at least one non-adhesive particle may be formulated in a composition comprising any cosmetically acceptable carrier, preferably one that does not substantially interfere with imparting at least one of the desirable properties such as, without limitation, reduced tack to the touch, luster, shine, silkier feel or appearance.

In addition, the at least one adhesive particle and at least one non-adhesive particle may be formulated in a composition comprising any cosmetically acceptable carrier that does not substantially interfere with substantially maintaining the reshapable effect.

Furthermore, the at least one adhesive particle and at least one non-adhesive particle may be formulated in a composition comprising any cosmetically acceptable carrier that does not substantially interfere with both imparting at least one of the desirable properties such as, without limitation, luster, shine, reduced tack to the touch, silkier feel or appearance and substantially maintaining the reshapable effect.

In one embodiment, the at least one adhesive particle and/or at least one non-adhesive particles are insoluble in the cosmetically acceptable carrier. In one embodiment, the at least one adhesive particle and/or at least one non-adhesive particles are present in a reshapable hair styling composition at a concentration ranging from about 0.1% to about 16.5% relative to the total weight of the composition.

The amount of adhesive particles may range up to about 15% by weight, more preferably up to about 10% by weight, even more preferably up to about 5% by weight and even more preferably up to about 3% by weight of the final composition. The non-adhesive particles will be provided in up to about 15% based on the weight of the adhesive particles, more preferably about 10% thereof or less.

One of ordinary skill in the art will choose the appropriate carrier based on the application envisaged. In one embodiment, the compositions of the invention may contain water; an organic solvent such as $C_1$ to $C_4$ alcohols including ethanol or isopropanol, $C_5$ to $C_{20}$ alkanes, acetone, methylethylacetone, methylacetate, ethylacetate, butylacetate, dimethoxyethane, and diethoxyethane; isododecane or mixtures thereof, such as a hydroalcoholic mixture. In one embodiment, the carrier may comprise an appropriate solvent to which may be added additives.

The compositions of the present invention may also comprise at least one additive known in the cosmetic arts that does not substantially interfere with imparting at least one of the desirable properties mentioned above and/or substantially maintaining the reshapable effect. At least one of such additives may be chosen from, but are not limited to: reducing agents (such as thiols); silanes (such as aminopropyl triethoxy silane); fatty substances; gelling agents; foaming agents; silicones; thickeners; anti-foaming agents; hydrating agents; fillers; sunscreens (such as UV filters); active haircare agents; perfumes; preservatives; cationic, anionic, nonionic, and amphoteric (such as zwitterionic) surfactants; cationic, anionic, nonionic, and amphoteric (such as zwitterionic) polymers; polyols; proteins; provitamins; vitamins; dyes; tints; bleaches; and pH adjusting agents. Examples of thickeners include cross-linked polyacrylic acids, such as Carbopol 980 from BF Goodrich Company. The compositions may also contain a conditioning agent such as, for example, such as silicone fluids, fatty esters, fatty alcohol, long chain hydrocarbons, emollients, lubricants, and penetrants such as lanolin compounds, protein hydrolysates, other protein derivatives, cationic and amphoteric polymers, and cationic surfactants. An example of a cationic silicone-conditioning agent is the product DC 939, sold by Dow Corning. These will be used in amounts which are conventional for the products contemplated and particularly those described or produced using adhesive particles alone.

The compositions according to the invention can be provided in any form known from the prior art, which is appropriate for their application to the hair, including products in the form of a vaporizable composition such as a spray or aerosol, mousse, gel, stick, mud, cream, foam, solution, dispersion or lotion. Indeed, the composition may be in any of the conventional forms of cosmetic composition including, but not limited to, shampoos, conditioners, hair rinses, permanent waving compositions, waving compositions, hair dye compositions, hair straightening compositions, hair fixing products, hair styling gel products, mousses, leave-on conditioners, spray products to be used before or after a hair dye treatment, products to be used before or after a permanent waving treatment, products to be used before or after a hair straightening treatment, and fixing foams.

In one embodiment, a reshapable hair styling composition comprises or consists essentially of at least one non-adhesive particle and at least one adhesive particle, as set forth above, wherein the at least one adhesive particle is present in an amount effective to provide a reshapable effect, the at least non-adhesive particle is present in an amount effective to impart at least one of the desirable properties such as, without limitation less tacky, less sticky, smoother, silkier feel or appearance of hair and/or substantially maintain the reshapable effect when compared to an identical formulation without the non-adhesive particles, and at least one carrier, the hair styling composition being in the form chosen from a spray, aerosol, mousse, gel, stick, mud, cream foam, solution, dispersion or lotion.

The composition according to the invention may be vaporizable, for example by a pump or may be a pressurized aerosol composition. It may be vaporizable by a dispensing valve controlled by a dispensing head, which in turn comprises

a nozzle, which vaporizes the aerosol composition. A vaporizable composition according to the invention comprises an appropriate vaporizable solvent. Advantageously, the appropriate vaporizable solvent comprises at least one solvent chosen from water and lower alcohols. In accordance with the invention, the term "lower alcohol" means a $C_1$ to $C_6$ aliphatic alcohol, such as ethanol and isopropanol.

When the vaporizable composition according to the invention is an aerosol composition, it additionally comprises an appropriate amount of propellant. The propellant comprises compressed or liquefied gases, which are normally employed for the preparation of aerosol compositions. Suitable gases include compressed air, carbon dioxide, nitrogen, and gases, which may be soluble in the composition such as dimethyl ether, halogenated hydrocarbons including fluorinated or non-fluorinated hydrocarbons, and mixtures thereof. In a preferred embodiment, the amount of propellant can range as high as 15% by weight of the total formulation. However, it has been found that these compositions can provide the advantages described herein even when they are applied as an aerosol and even when they are applied using a reduced level of propellant, i.e. 10% or less, preferable about 7% or less and more preferably about 6% or less.

One embodiment of the present invention additionally provides an aerosol device comprising a vessel comprising an aerosol composition, which comprises a liquid phase (or juice) comprising at least one hair styling composition comprising at least one non-adhesive particle, at least one adhesive particle as described above, in a cosmetically acceptable carrier and optionally a propellant. The device includes a dispenser, such as a dispensing valve or pump for dispensing the aerosol composition from the vessel. In one embodiment, the at least one adhesive particle is present in an amount effective to provide a reshapable effect, and the at least one non-adhesive particle is present in an amount effective to impart at least one of the desirable properties such as, without limitation less tacky, less sticky, smoother, silkier feel or appearance of hair and/or substantially maintain the reshapable effect when compared to an identical formulation without the non-adhesive particles as judged by styling professionals.

The present invention also relates to a method of providing a reshapable effect to the hair comprising applying to the hair, a hair styling composition that comprises, optionally in a cosmetically acceptable carrier, at least one non-adhesive particle and at least one adhesive particle, as set forth above, wherein the at least one adhesive particle is present in an amount effective to provide a reshapable effect, and the at least one non-adhesive particle is present in an effective amount to impart at least one of the desirable properties such as, without limitation less tacky, less sticky, smoother, silkier feel or appearance of hair and/or substantially maintain the reshapable effect when compared to an identical formulation without the non-adhesive particles. In addition, the invention is drawn to a method of imparting at least one of the desirable properties to the hair comprising applying to the hair a reshapable hair styling composition that comprises, optionally in a cosmetically acceptable carrier, at least one non-adhesive particle and at least one adhesive particle, as set forth above, wherein the at least one adhesive particle is present in an amount effective to provide a reshapable effect, and the at least one non-adhesive particle to impart at least one of the desirable properties such as, without limitation less tacky, less sticky, smoother, silkier feel or appearance of hair and/or substantially maintain the reshapable effect when compared to an identical formulation without the non-adhesive particles.

Another embodiment of the present invention additionally provides a method of cosmetically treating keratinous fibers, especially hair, comprising applying to the hair before, during, or after the shaping of the hairstyle, a hair styling composition comprising at least one non-adhesive particle and at least one adhesive particle, as set forth above. In one embodiment, the at least one adhesive particle is present in an effective amount to provide a reshapable effect, and the at least one non-adhesive particle is present in an effective amount to impart at least one of the desirable properties such as, without limitation less tacky, less sticky, smoother, silkier feel or appearance of hair and/or substantially maintain the reshapable effect when compared to an identical formulation without the non-adhesive particles.

A further embodiment of the present invention provides a method of manufacturing a hair styling composition comprising at least one non-adhesive particle and at least one adhesive particle, as set forth above. In one embodiment, the at least one adhesive particle is present in an amount effective to provide a reshapable effect, and the at least one non-adhesive particle is present in an amount effective to impart at least one of the desirable properties such as, without limitation, less tacky, less sticky, smoother, silkier feel or appearance of hair and/or substantially maintain the reshapable effect when compared to an identical formulation without the non-adhesive particles.

In yet a further embodiment, the present invention provides a reshapable hair composition comprising or consisting essentially of up to about 16.5% total of adhesive and non-adhesive particles, up to about 15% of at least one adhesive particle by weight based on the weight of the composition, up to about 12% of at least one non-adhesive particle by weight of the adhesive particles and at least one carrier.

In yet a further embodiment, the present invention provides a reshapable hair composition comprising or consisting essentially of up to about 16.5% total of adhesive and inorganic non-adhesive particles, from about 0.05% to about 15% of at least one adhesive particle by weight based on the weight of the composition, from about 0.2% to about 12% of at least one inorganic non-adhesive particle by weight of the adhesive particles and at least one carrier.

PREPARATION OF A HAIR STYLING MOUSSE

The following formulation (Example 1) comprised only the at least one adhesive particle. A styling mousse was formulated by mixing the mixture of ingredients and the propellant. Typically, the mousse formula comprises 90% of ingredients and 10% of propellant A-46. The mixture of ingredients was prepared by mixing Gel-Tac 205D (adhesive particles), non-adhesive polymer particles, Empicol BSD 52 and water under rigorous agitation until they are mixed well. (See examples 13 and 14.) The final pH of the mixture was adjusted within 6.0-7.0. The resulting mousse composition was then applied to hair tresses as well as hairs on mechanical heads. The comparison was made against the original mousse formula without the non-adhesive particles, Gel-Tac 205D, which is demonstrated in Example 1. All ingredients amounts are shown in weight percentages.

Example 1

| Ingredient | Particle size | Percentage |
|---|---|---|
| Gel-Tac 205D (adhesive) | 20 µm | 1.80% |
| Empicol BSD 52 | | 0.18% |
| Water | | 88.02% |
| Propellant A-46 | | 10.00% |

Example 2

| Ingredient | Particle size | Percentage |
|---|---|---|
| Gel-Tac 205D (adhesive) | 20 µm | 1.88% |
| Tegobetaine HS | | 0.47% |
| Brij 30 | | 0.47% |
| Water | | 91.18% |
| Propellant A-46 | | 6.00% |

The preparation for Example 2 is the same as that of making Example 1, except the propellant level was 6%. Example 2 and Example 1 gave the same performance results in salon testing on the head of a subject. With 6% propellant level, the formula is more suitable for commercial application as the new regulation demands low VOC level.
The following five formulations comprising at least one non-adhesive particle that is constituted of a single polymeric material and at least one adhesive particle (Examples 3-12) were prepared and tested against the original mousse formulation. The resulting compositions of Examples 3-12 provided unsatisfactory results. Upon application to hair, the hair felt greasy, tacky, and sticky and lacked luster, just like an identical formulation without the at least one non-adhesive particle of Example 1.

Example 3

| Ingredient | Particle size | Percentage |
|---|---|---|
| Gel-Tac 205D | 20 µm | 1.80% |
| Poly(methyl methacrylate) cross-linked | 8µm | 0.09% |
| Empicol BSD 52 | | 0.18% |
| Water | | 87.93% |
| Propellant A-46 | | 10.00% |

Example 4

| Ingredient | Particle size | Percentage |
|---|---|---|
| Gel-Tac 205D | 20 µm | 1.17% |
| Poly(methyl methacrylate) cross-linked | 15µm | 0.09% |
| Empicol BSD 52 | | 0.18% |
| Water | | 88.56% |
| Propellant A-46 | | 10.00% |

Example 5

| Ingredient | Particle size | Percentage |
|---|---|---|
| Gel-Tac 205D | 20 µm | 1.17% |
| Poly(methyl methacrylate) cross-linked | 30µm | 0.09% |
| Empicol BSD 52 | | 0.18% |
| Water | | 88.56% |
| Propellant A-46 | | 10.00% |

Example 6

| Ingredient | Particle size | Percentage |
|---|---|---|
| Gel-Tac 205D | 20 µm | 1.17% |
| Poly(methyl methacrylate) cross-linked | 40µm | 0.09% |
| Empicol BSD 52 | | 0.18% |
| Water | | 88.56% |
| Propellant A-46 | | 10.00% |

Example 7

| Ingredient | Particle size | Percentage |
|---|---|---|
| Gel-Tac 205D | 20 µm | 1.17% |
| Poly(methyl methacrylate) non cross-linked | 4µm | 0.09% |
| Empicol BSD 52 | | 0.18% |
| Water | | 88.56%% |
| Propellant A-46 | | 10.00 % |

Example 8

| Ingredient | Particle size | Percentage |
|---|---|---|
| Gel-Tac 205D | 20 µm | 1.17% |
| Poly(methyl methacrylate) non cross-linked | 8µm | 0.09 |
| Empicol BSD 52 | | 0.18% |
| Water | | 88.56% |
| Propellant A-46 | | 10.00% |

Example 9

| Ingredient | Particle size | Percentage |
|---|---|---|
| Gel-Tac 205D | 20 µm | 1.80% |
| Poly(styrene) cross-linked | 8µm | 0.09% |
| Empicol BSD 52 | | 0.18% |
| Water | | 87.93% |
| Propellant A-46 | | 10.00% |

Example 10

| Ingredient | Particle size | Percentage |
|---|---|---|
| Gel-Tac 205D | 20 µm | 1.80% |
| Poly(acrylic ester) cross-linked | 15µm | 0.10% |
| Empicol BSD 52 | | 0.18% |
| Water | | 87.92% |
| Propellant A-46 | | 10.00% |

Example 11

| Ingredient | Particle size | Percentage |
|---|---|---|
| Gel-Tac 205D | 20 µm | 1.80% |
| Polymethyl methacrylate cross-linked; aspect ratio 1.2-1.3 | 3µm 12µm | 0.09% |
| Empicol BSD 52 | | 0.18% |
| Water | | 87.93% |
| Propellant A-46 | | 10.00% |

Example 12

| Ingredient | Particle size | Percentage |
|---|---|---|
| Gel-Tac 205D | 20 µm | 1.80% |
| Polymethyl methacrylate cross-linked; aspect ratio 1.5-1.8 | 3µm 12µm | 0.09% |
| Empicol BSD 52 | | 0.18% |
| Water | | 87.93% |
| Propellant A-46 | | 10.00% |

The following three formulations (Examples 13-15), upon application to hair, demonstrated at least one improved cosmetic property such as non-greasy, less sticky, less tacky, silkier and drier feel with reshapable effect intact compared to an identical formulation without the at least one non-adhesive particle produced in accordance with Example 1.

Example 13

| Ingredient | Particle size | Percentage |
|---|---|---|
| Gel-Tac 205D | 20 µm | 1.80% |
| Core/shell (polystyrene as the core & polymethyl methacrylate as the shell ) cross-linked | 8µm | 0.09% |
| Empicol BSD 52 | | 0.18% |
| Water | | 87.93% |
| Propellant A-46 | | 10.00% |

Example 14

| Ingredient | Particle size | Percentage |
|---|---|---|
| Gel-Tac 205D | 20 µm | 1.80% |
| Polystyrene cross-linked | 8µm | 0.07% |
| Polymethyl methacrylate cross-linked (1:1 blend) | 8µm | 0.07% |
| Empicol BSD 52 | | 0.18% |
| Water | | 87.88% |
| Propellant A-46 | | 10.00% |

Example 15

| Ingredient | Particle size | Percentage |
|---|---|---|
| Gel-Tac 205D | 20 µm | 1.80% |
| Core/shell (polystyrene as the core & polymethyl methacrylate as the shell ) cross-linked | 8µm | 0.09% |
| Empicol BSD 52 | | 0.18% |
| Water | | 91.93% |
| Propellant A-46 | | 6.00% |

The composition in Example 15 was produced as described previously for Examples 1 and 2.

Example 16

| Ingredient | Particle size | Percentage |
|---|---|---|
| Gel-Tac 205D | 20 µm | 1.84% |
| Poly(acrylic acid) cross-linked | 15µm | 0.16% |
| SynthalenK | | 1.00% |
| Water | | 97% |

This example demonstrates that a successful gel composition can be made with a single type of polymer particle.

The following four mousse formulations (Examples 17-20), upon application to hair, demonstrated improvement in at least one cosmetic property such as non-greasy, less sticky, less tacky, silkier and drier feel with reshapable effect intact compared to an identical formulation without the at least one inorganic non-adhesive particle produced in accordance with Example 1.

Example 17

| Ingredient | Particle size | Percentage |
|---|---|---|
| Gel-Tac® 205D | 20µm | 1.17% |
| Luxsil® | 9µm | 0.06% |
| Empicol BSD 52 | - | 0.18% |
| Water | - | 88.59% |
| Propellant A-46 | - | 10.00% |

Example 18

| Ingredient | Particle size | Percentage |
|---|---|---|
| Gel-Tac® 205D | 20µm | 1.76% |
| Biron® Liquid Silver | 15µm | 0.04% |
| Empicol BSD 52 | - | 0.18% |
| Water | - | 88.02% |
| Propellant A-46 | - | 10.00% |

Example 19

| Ingredient | Particle size | Percentage |
| --- | --- | --- |
| Gel-Tac® 205D | 20µm | 1.71% |
| Xirona® Nordic Sunset | 19.3µm | 0.09% |
| Empicol BSD 52 | - | 0.18% |
| Water | - | 88.02% |
| Propellant A-46 | - | 10.00% |

Example 20

| Ingredient | Particle size | Percentage |
| --- | --- | --- |
| Gel-Tac® 205D | 20µm | 1.17% |
| Timiron® Diamond Cluster MP149 | 72.3µm | 0.06% |
| Empicol BSD 52 | - | 0.18% |
| Water | - | 88.59% |
| Propellant A-46 | - | 10.00% |

The following three gel formulations (Examples 21-23), upon application to hair, demonstrated improvement in at least one cosmetic property such as non-greasy, less sticky, less tacky, silkier and drier feel with reshapable effect intact compared to an identical formulation without the at least one inorganic non-adhesive particle produced in accordance with Example 14.

Example 21

-

| Ingredient | Particle size | Percentage |
| --- | --- | --- |
| Gel-Tac® 100G | 15µm | 1.90% |
| Omyapure® 35 | 2µm | 0.15% |
| Synthalen K | - | 1.00% |
| Water | - | 95.85% |

Example 22

| Ingredient | Particle size | Percentage |
| --- | --- | --- |
| Gel-Tac® 100G | 15µm | 1.90% |
| Luxsil® | 9µm | 0.10% |
| Synthalen K | - | 1.00% |
| Water | - | 97.00% |

Example 23

| Ingredient | Particle size | Percentage |
|---|---|---|
| Gel-Tac® 100G | 15µm | 1.90% |
| Timiron® Diamond Cluster MP 149 | 72.3µm | 0.10% |
| Synthalen K | - | 1.00% |
| Water | - | 97.00% |

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A hair styling composition comprising: a mixture of an effective amount of at least one adhesive particle of up to about 15% by weight of said composition; an effective amount of at least one polymeric non-adhesive particle selected from a single type of particle, a blend of different particles and a core/shell particle provided in an amount of up to about 10% by weight of said composition; and at least one cosmetically acceptable carrier, said hair styling composition substantially maintaining a reshapable effect.

2. The hair styling composition of claim 1, wherein said at least one non-adhesive particle has an average particle size from 6 to 30 µm and wherein the ratio of said adhesive to said non-adhesive particles ranges from 2:1 to 50:1.

3. The hair styling composition of claim 1 or 2, wherein said at least one non-adhesive particle is present in an amount of between 0.03 and 2.0% by weight of said composition.

4. The hair styling composition of claim 3, wherein said at least one non-adhesive particle is present in an amount of between 0.04 and 0.3% by weight of said composition.

5. The hair styling composition of any preceding claim, wherein said at least one non-adhesive particle has an average particle size from 7 to 25 µm.

6. The hair styling composition of any preceding claim, exhibiting acceptable results in terms of greasiness, tackiness, stickiness, smoothness or silky feel when compared to the identical composition without an effective amount of said polymeric non-adhesive particles.

7. The hair styling composition of any preceding claim, wherein the shell of said core/shell structure comprises a material which is more hydrophilic than the material used for the core thereof.

8. The hair styling composition of any preceding claim, wherein said core comprises a styrene resin.

9. The hair styling composition of claim 8, wherein said styrene resin comprises an alkyl styrene, halogenated styrene, nitrostyrene, acetylstyrene, or methoxystyrene.

10. The hair styling composition of claim 9, wherein said alkyl styrene comprises methylstyrene, dimethylstyrene, trimethylstyrene, ethylstyrene, diethylstyrene, triethylstyrene, propylstyrene, butylstyrene, hexylstyrene, heptylstyrene, or octylstyrene.

11. The hair styling composition of claim 10, wherein said halogenated styrene comprises fluorostyrene, chlorostyrene, bromostyrene, dibromostyrene, iodostyrene, or chloromethylstyrene.

**12.** The hair styling composition of any preceding claim, wherein said core comprises cross-linked polystyrene.

**13.** The hair styling composition of any preceding claim, wherein said shell comprises a (meth)acrylate-based resin.

**14.** The hair styling composition of claim 13, wherein said shell comprises methyl (meth)acrylate, ethyl (meth)acrylate, propyl (meth)acrylate, butyl (meth)acrylate, 2-ethylhexyl (meth)acrylate, lauryl (meth)acrylate, stearyl (meth) acrylate, cyclohexyl (meth)acrylate, 2-hydroxyethyl (meth)acrylate, 2-propyl (meth)acrylate, chloro-2-hydroxyethyl (meth)acrylate, diethylene-glycol mono(meth)acrylate, methoxy ethyl (meth)acrylate, glycidyl (meth)acrylate, dicyclopentanyl (meth)acrylate, or isobronol (meth)acrylate.

**15.** The hair styling composition of any preceding claim, wherein said shell comprises cross-linked polymethyl methacrylate.

**16.** The hair styling composition of any preceding claim, wherein said at least one non-adhesive particle comprises a blend of two or more particles of different polymers.

**17.** The hair styling composition of claim 16, wherein said different polymers comprises styrene resins, methacrylate-based resins, polyamide-based resins, polyolefine-based resins, cellulose-based resins, silicone-based resins, or fluorine-based resins.

**18.** The hair styling composition of claim 16, wherein said blend of two or more particles of said different polymers comprises a blend of a styrene-based resin and a methacrylate-based resin.

**19.** The hair styling composition of claim 18, wherein said blend of two or more particles of said different polymers comprises a blend of polystyrene and polymethyl methacrylate.

**20.** The hair styling composition of any of claims 16 to 19, wherein said blend of two or more particles of said different polymers has a ratio of one polymer to other polymer from 2.5:1 to 1:2.5.

**21.** The hair styling composition of claim 20, wherein said blend of two or more particles of said different polymers has a ratio of one polymer to other polymer from 2:1 to 1:2.

**22.** The hair styling composition of claim 21, wherein said blend of two or more particles of said different polymers has a ratio of one polymer to other polymer from 1.5:1 to 1:1.5.

**23.** The hair styling composition of claim 23, wherein said blend of two or more particles of different polymers has a ratio of one polymer to other polymer about 1:1.

**24.** The hair styling composition of any preceding claim, wherein said carrier comprises water, C1-C4 alcohol, C5-C20 alkane, acetone, isododecane, methylethylacetone, methylacetate, ethylacetate, butylacetate, dimethoxyethane, diethoxyethane, or mixtures thereof.

**25.** The hair styling composition of any preceding claim, further comprising at least one additive.

**26.** The hair styling composition of claim 25, wherein said at least one additive comprises a conditioning agent, reducing agent, silane, fatty substance, thickener, anti-foaming agent, hydrating agent, filler, sunscreen, active hair care agent, perfume, preservative, cationic, anionic, nonionic, and amphoteric surfactant, cationic, anionic, nonionic and amphoteric polymer, polyol, protein, provitamin, vitamin, dye, tint, bleach, or pH adjusting agent.

**27.** The hair styling composition of any preceding claim, wherein said hair styling composition is in the form of a spray, aerosol, mousse, gel, stick, mud or lotion.

**28.** The hair styling composition of any preceding claim, wherein said hair styling composition is a shampoo, conditioner, hair rinse, permanent waving composition, waving composition, hair dye composition, hair straightening composition, hair fixing product, hair styling product, hair spray, hair gel, hair mousse, or leave-on conditioner.

**29.** A method of cosmetically treating hair and/or of providing a re-shapeable effect to the hair, comprising applying to the hair before, during, or after shaping of a hairstyle, a hair styling composition of any preceding claim 3.

**30.** A method of both providing a reshapable effect and imparting acceptable results in terms of greasiness, tackiness, stickiness, smoothness or silky feel when compared to the identical composition without an effective amount of said polymeric non-adhesive particles to said hair, comprising applying to the hair, before, during or after shaping of the hairstyle, a hair styling composition of any of claims 1 to 28.

**31.** An aerosol hair styling composition comprising: 6% or less of a propellant based on the weight of said composition and a hair styling composition of any of claims 1 to 28.

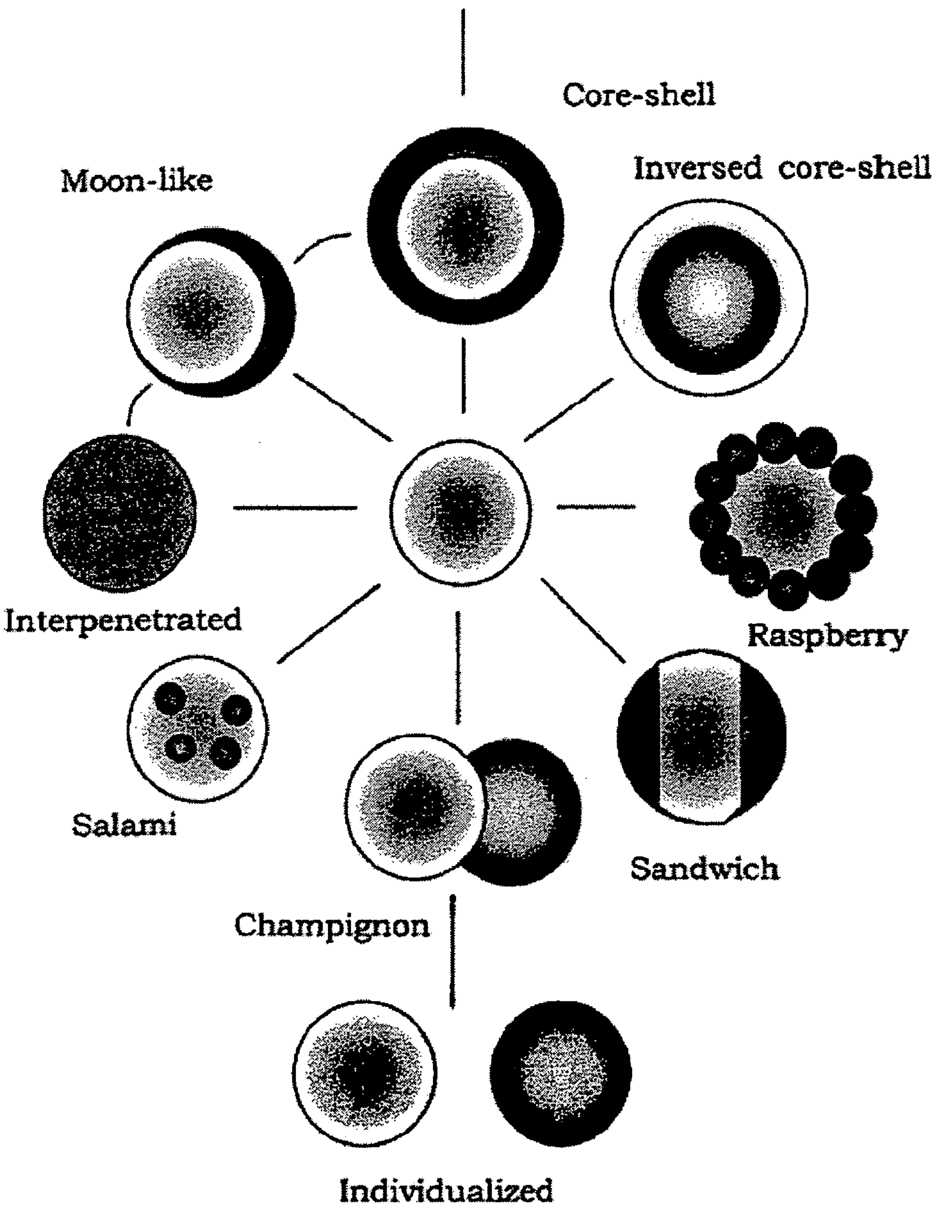
Core-shell
Moon-like
Inversed core-shell
Interpenetrated
Raspberry
Salami
Sandwich
Champignon
Individualized

# FIGURE 1

European Patent Office

# EUROPEAN SEARCH REPORT

Application Number
EP 05 25 4784

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 149 898 A (PEFFLY ET AL) 21 November 2000 (2000-11-21) * column 3, line 2; examples IV-XVIII * | 1-31 | A61K7/06 |
| X | US 2003/143180 A1 (GIROUD FRANCK ET AL) 31 July 2003 (2003-07-31) * claim 8; example c * | 1-31 | |
| X | US 5 120 532 A (WELLS ET AL) 9 June 1992 (1992-06-09) * examples III-V * | 1-31 | |
| X | US 6 592 854 B1 (DUPUIS CHRISTINE) 15 July 2003 (2003-07-15) * examples 1-8 * | 1-31 | |
| X | US 6 517 821 B1 (ROLLAT ISABELLE ET AL) 11 February 2003 (2003-02-11) * claim 1; examples 15,16 * | 1-31 | |
| X | EP 0 797 979 A (L'OREAL) 1 October 1997 (1997-10-01) * examples 1-6 * | 1-31 | TECHNICAL FIELDS SEARCHED (Int.Cl.7) A61K |

The present search report has been drawn up for all claims

| Place of search | Date of completion of the search | Examiner |
|---|---|---|
| Munich | 9 November 2005 | Vayssié, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 05 25 4784

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2005

| Patent document cited in search report | | Publication date | Patent family member(s) | | | Publication date |
|---|---|---|---|---|---|---|
| US 6149898 | A | 21-11-2000 | AU | 3950099 | A | 10-01-2000 |
| | | | BR | 9911504 | A | 17-04-2001 |
| | | | CA | 2331138 | A1 | 29-12-1999 |
| | | | CN | 1306414 | A | 01-08-2001 |
| | | | EP | 1087746 | A1 | 04-04-2001 |
| | | | WO | 9966888 | A1 | 29-12-1999 |
| | | | JP | 2002518423 | T | 25-06-2002 |
| US 2003143180 | A1 | 31-07-2003 | NONE | | | |
| US 5120532 | A | 09-06-1992 | AR | 247482 | A1 | 31-01-1995 |
| | | | AT | 161417 | T | 15-01-1998 |
| | | | AU | 7661891 | A | 30-10-1991 |
| | | | BR | 9106318 | A | 20-04-1993 |
| | | | CA | 2079680 | A1 | 07-10-1991 |
| | | | CN | 1056416 | A | 27-11-1991 |
| | | | CS | 9100974 | A3 | 19-02-1992 |
| | | | DE | 69128529 | D1 | 05-02-1998 |
| | | | DE | 69128529 | T2 | 30-07-1998 |
| | | | EP | 0523109 | A1 | 20-01-1993 |
| | | | ES | 2110992 | T3 | 01-03-1998 |
| | | | FI | 924473 | A | 05-10-1992 |
| | | | HU | 62450 | A2 | 28-05-1993 |
| | | | IE | 911139 | A1 | 09-10-1991 |
| | | | JP | 3247107 | B2 | 15-01-2002 |
| | | | JP | 5508389 | T | 25-11-1993 |
| | | | MA | 22116 | A1 | 31-12-1991 |
| | | | NZ | 237701 | A | 28-04-1992 |
| | | | PT | 97273 | A | 31-12-1991 |
| | | | TR | 26319 | A | 15-03-1995 |
| | | | WO | 9115185 | A1 | 17-10-1991 |
| US 6592854 | B1 | 15-07-2003 | AT | 229313 | T | 15-12-2002 |
| | | | AU | 732427 | B2 | 26-04-2001 |
| | | | AU | 4952197 | A | 03-06-1998 |
| | | | BR | 9714493 | A | 06-06-2000 |
| | | | CA | 2270591 | A1 | 22-05-1998 |
| | | | CN | 1244789 | A | 16-02-2000 |
| | | | DE | 69717855 | D1 | 23-01-2003 |
| | | | DE | 69717855 | T2 | 28-05-2003 |
| | | | EP | 0948310 | A2 | 13-10-1999 |
| | | | ES | 2188920 | T3 | 01-07-2003 |
| | | | FR | 2755608 | A1 | 15-05-1998 |
| | | | WO | 9820833 | A2 | 22-05-1998 |
| | | | JP | 2000504346 | T | 11-04-2000 |
| | | | JP | 2004307520 | A | 04-11-2004 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 25 4784

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2005

| Patent document cited in search report | Publication date | Patent family member(s) | Publication date |
|---|---|---|---|
| US 6592854 B1 | | KR 2000053125 A | 25-08-2000 |
| | | PL 333271 A1 | 22-11-1999 |
| US 6517821 B1 | 11-02-2003 | AU 8001601 A | 13-02-2002 |
| | | EP 1307176 A2 | 07-05-2003 |
| | | WO 0209654 A2 | 07-02-2002 |
| | | JP 2004517809 T | 17-06-2004 |
| EP 0797979 A | 01-10-1997 | AT 170739 T | 15-09-1998 |
| | | AU 704016 B2 | 01-04-1999 |
| | | AU 1773697 A | 11-12-1997 |
| | | BR 9700517 A | 03-11-1998 |
| | | CA 2201958 A1 | 05-10-1997 |
| | | CN 1172640 A | 11-02-1998 |
| | | DE 69700024 D1 | 15-10-1998 |
| | | DE 69700024 T2 | 28-01-1999 |
| | | ES 2124638 T3 | 01-02-1999 |
| | | FR 2747036 A1 | 10-10-1997 |
| | | JP 2941227 B2 | 25-08-1999 |
| | | JP 10036234 A | 10-02-1998 |
| | | KR 236859 B1 | 01-02-2000 |
| | | PL 319290 A1 | 13-10-1997 |
| | | RU 2132674 C1 | 10-07-1999 |

EPO FORM P0459

For more details about this annex : see Official Journal of the European Patent Office, No. 12/82